# EUROPEAN PATENT APPLICATION

(11) **EP 3 156 925 A2**
(43) Date of publication of application: **19.04.2017**
(21) Application number: 16184336.2
(22) Date of filing: 27.02.2009
(51) Int. Cl.: G06F 19/24

(54) **COPD BIOMARKER SIGNATURES**

(30) Priority: 10.03.2008 US 68772 P
(62) Divisional of application: 09718959.1
(71) Applicant: Lineagen, Inc., Salt Lake City, UT 84108 (US)
(72) Inventor: GERVAIS, Francois, Kirkland, Québec H9H 3L1 (CA); DEVANARAYAN, Viswanath, Souderton, PA 18964 (US)
(74) Representative: Kehoe, Laura Ellen

(57) **Abstract**

The present invention relates to methods of detecting differentially expressed protein expression indicative of COPD in a test sample. The detection of circulating levels of proteins within an identified COPD biomarker signature can aid in COPD diagnosis and disease monitoring, as well as in the prediction of responses to therapeutics. Evaluation of the biomarker signatures disclosed, or a subset of biomarkers thereof, provides a level of discrimination not found with individual markers.

## Description

### CROSS-REFERENCE TO RELATED APPLICATIONS

The present application claims the benefit of U.S. Provisional Application No. 61/068,772, filed March 10, 2008, hereby incorporated by reference herein.

### FIELD OF THE INVENTION

The present invention relates generally to methods for the rapid detection and accurate diagnosis of chronic obstructive pulmonary disease (COPD). More specifically, it relates to biomarker signatures indicative of COPD and detecting the differential expression of one or more proteins within the biomarker signatures to classify a test sample.

### BACKGROUND OF THE INVENTION

Chronic obstructive pulmonary disease (COPD) is defined by a progressive airflow limitation caused by an abnormal inflammatory reaction to the inhalation of particles such as cigarette smoke (for a review, see Fabbri et al., 2006, Am. J. Respir. Crit. Care Med. 173:1056-1065). It is a leading cause of death in the US (Heffner, 2002, Respir. Care 47:586-607). Doctors diagnose COPD by observing a patient's symptoms, evaluating life-style choices such as smoking and occupation, performing physical examinations, and conducting spirometry tests to measure patient's airflow. The gold standard diagnostic parameter of COPD is declining FEV1 (forced expiratory volume in 1 second) measured by spirometry (Rennard, 1998, Chest 113:235S-241S). In normal non-smokers and smokers, a slow but progressive decline in FEV1 is observed (20 ml/year). In about 15% of smokers, lung function declines more rapidly than normal, leading to an accelerated limitation in physical performance (poor exercise tolerance) and dyspnea (Rennard, 1998, *supra*)*.* The rate of decline between COPD patients can vary greatly.

U.S. Patent Application Publication No. US 2006/0211026 (published September, 2006 in the names of P. Belloni et al.) discloses methods for diagnosing emphysema and COPD and assessing the efficacy of therapeutic drug candidates by determining if the relative level of a biomarker in a biological sample is higher or lower than the expected level, wherein the biomarker is selected from the group consisting of specific SpB, desmosine, VEGF, IGFBP2, MMP12, TIMP1, MMP9, Crabp2, Rbp1, Cyp26a1, Tgm2, Timp3, Adam17, Serpina1, Slpi, Col1al1, Eln, TGF 1, TGF -RII, Sftpa1, Sftpb, Csf2, Cxcl1, Cxcl2, Cxcl5, IL-8R , IL-8R , IL-6, TNF, EGF-R, Areg, PDGF , HpGF, FGF7, Kdr, flt1, Angpt1, Tek, HIF1 , Hyou1, PGF, and tropoelastin.

PCT Application Publication No. WO 2004/070058 (published August 19, 2004 in the name of Bayer Healthcare AG) discloses the overexpression of human membrane spanning 4-domains, subfamily S, member 8B ("MS4A8B") in COPD patients and its use as a diagnostic and prognostic marker for COPD.

PCT Application Publication No. WO 2006/118522 (published November 9, 2006 in the name of Astrazeneca AB) discloses the identification and use of peptides derived from zinc- 2-glycoprotein, 1-antitrypsin, collagen type III, prostaglandin-H2 D isomerase, collagen type I, 1-microglobulin, FGF, osteopontin, 1-acid glycoprotein 2 and fibrinogen alpha-E chain as biomarkers for COPD.

PCT Application Publication No. WO 2007/084485 (published July 26, 2007 in the name of Batelle Memorial Institute) identifies a variety of gene markers differentially expressed in smokers and the use of said markers to assess COPD-related diseases.

PCT Application Publication No. WO 2008/003066 (published January 3, 2008 in the name of Respiris, Inc.) discloses methods of identifying COPD markers from informative content repositories and the use of said markers to diagnose COPD, including assessing disease progression among rapid and slow decline conditions.

### SUMMARY OF THE INVENTION

Methods for the rapid detection and/or accurate diagnosis of chronic obstructive pulmonary disease (COPD) are provided. The methods can be practiced by quantifying at least a subset of protein biomarkers contained within one of three disclosed multi-analyte panels in a test sample (e.g., blood or blood derivative, such as plasma or serum). Altered levels of the selected biomarkers which are statistically different from levels found in control subjects support a positive diagnosis of COPD. Thus, the multi-analyte panels of protein biomarkers, described herein as "biomarker signatures," found in plasma samples of patients diagnosed with COPD help to support a positive or negative diagnosis of COPD, as well as to classify the severity of a positive COPD diagnosis (e.g., rapidly declining COPD and slowly declining COPD). In certain embodiments, greater than 90% accuracy in making a correct diagnosis and/or classification is provided by the disclosed methods.

Circulating proteins are identified and described herein that are differentially expressed in COPD patients. The circulating plasma markers include apolipoprotein H, CD40, haptoglobin, interleukin-8 ("IL-8"), monocyte chemoattractant protein-1 ("MCP-1"), tumor necrosis factor receptor II ("TNF-RII"), apolipoprotein CIII, granulocyte-macrophage colony stimulating factor ("GM-CSF"), immunoglobulin A ("IgA"), macrophage inflammatory protein 1-alpha ("MIP-1 "), tissue factor, tumor necrosis factor-alpha ("TNF- "), alpha-1 antitrypsin, C-reactive protein ("CRP"), fibrinogen, interleukin-4 ("IL-4"), macrophage-derived chemokine ("MDC"), and soluble vascular cell adhesion molecule 1 ("sVCAM-1"). Three biomarker signatures comprising a subset of the plasma markers listed above are disclosed which enable one to distinguish between (a) rapidly declining COPD patients and controls (Signature 1), (b) slowly declining COPD patients and controls (Signature 2), and (c) rapidly declining COPD patients and slowly declining COPD patients (Signature 3).

Thus, the present invention provides methods of detecting differentialprotein expression indicative of COPD in a test sample, wherein said test sample includes but is not limited to blood or a blood derivative (e.g., plasma). The detection of circulating levels of proteins identified herein can classify patients as having COPD (diagnosis), as well as classify patients diagnosed with COPD according to the severity of the disease (disease monitoring). Such classification can be used in prediction of a response to a therapeutic to aid in the development of successful therapies to treat COPD and to help provide an early assessment of drug efficacy in clinical trials *(i.e.,* following therapeutic regimens in patients). For example, in a single time point or time course, measurement of expression of the disclosed biomarkers can be determined after a patient has been exposed to a therapy, which may include, for example, drug therapy, combination drug therapy and non-pharmacologic intervention. Evaluation of the biomarker signatures disclosed herein, or a subset of biomarkers thereof, provides a level of discrimination not found with individual markers. In one embodiment, the expression profile is determined by measurement of protein concentrations or amounts.

In one embodiment, the present invention provides for a computer-implemented method of classifying a test sample obtained from a mammalian subject, comprising (a) obtaining a dataset associated with said test sample, wherein said obtained dataset *(i.e.,* test dataset) comprises quantitative data for at least three protein markers selected from a multi-analyte panel selected from the group consisting of: (i) apolipoprotein H, CD40, haptoglobin, interleukin-8 (IL-8), monocyte chemoattractant protein-1 (MCP-1) and tumor necrosis factor receptor II (TNF-RII); (ii) apolipoprotein CIII, CD40, granulocyte-macrophage colony stimulating factor (GM-CSF), haptoglobin, immunoglobulin A (IgA), macrophage inflammatory protein 1alpha (MIP-1), tissue factor and tumor necrosis factor-alpha (TNF- ); and, (iii) alpha-1 antitrypsin, C-reactive protein (CRP), fibrinogen, granulocyte-macrophage colony stimulating factor (GM-CSF), interleukin-4 (IL-4), macrophage-derived chemokine (MDC), tissue factor, tumor necrosis factor receptor II (TNFRII) and soluble vascular cell adhesion molecule 1 (sVCAM-1); (b) inputting said obtained dataset into an analytical process on a computer that compares said obtained dataset against one or more reference datasets; and, (c) classifying said sample according to the output of said analytical process, wherein said classification is selected from the group consisting of a chronic obstructive pulmonary disease (COPD) classification and a healthy classification. A preferred mammalian subject is a human subject.

In a further embodiment, the present invention relates to a method for classifying a test sample obtained from a human subject, comprising: (a) obtaining a dataset associated with said test sample, wherein said obtained dataset comprises quantitative data for at least three protein markers selected from a multi-analyte panel selected from the group consisting of: (i) apolipoprotein H, CD40, haptoglobin, interleukin-8 (IL-8), monocyte chemoattractant protein-1 (MCP-1) and tumor necrosis factor receptor II (TNF-RII); (ii) apolipoprotein CIII, CD40, granulocyte-macrophage colony stimulating factor (GM-CSF), haptoglobin, immunoglobulin A (IgA), macrophage inflammatory protein 1 alpha (MIP-1 ), tissue factor and tumor necrosis factor-alpha (TNF- ); and, (iii) alpha-1 antitrypsin, C-reactive protein (CRP), fibrinogen, granulocyte-macrophage colony stimulating factor (GM-CSF), interleukin-4 (IL-4), macrophage-derived chemokine (MDC), tissue factor, tumor necrosis factor receptor II (TNFRII) and soluble vascular cell adhesion molecule 1 (sVCAM-1); (b) transforming said obtained dataset to classify said test sample using an analytical process that compares said obtained dataset against one or more reference datasets; and, (c) classifying said test sample according to the output of said analytical process, wherein said classification is selected from the group consisting of a chronic obstructive pulmonary disease (COPD) classification and a healthy classification.

Methods of analysis disclosed herein include, without limitation, utilizing one or more reference datasets to generate a predictive model. Test sample data is compared to the predictive model in order to classify the sample, wherein said classification is selected from the group consisting of a COPD classification and a healthy classification. A COPD classification can represent a rapidly declining COPD classification or a slowing declining COPD classification.

A predictive model of the invention utilizes quantitative data of one or more sets of markers described herein obtained from a reference population. In one embodiment, the quantitative data represents protein concentrations or relative amounts of the protein biomarkers (e.g., as measured via a suitable detection method) within the disclosed biomarker signatures, or a subset of protein biomarkers thereof, in blood or a blood derivative (e.g., plasma). A predictive model can provide for a level of accuracy in classification wherein the model satisfies a desired quality threshold. A quality threshold of interest may provide for an accuracy of a given threshold and may be referred to herein as a quality metric. A predictive model may provide a quality metric, e.g. accuracy of classification, of at least about 70%, at least about 80%, at least about 90%, or higher. Within such a model, parameters may be appropriately selected so as to provide for a desired balance of sensitivity and selectivity.

In one embodiment of the present invention, such a predictive model can be used in methods to classify a test sample obtained from a mammalian subject as being derived from a healthy individual or an individual with COPD, in particular rapidly declining COPD. A first step in said method is to obtain a dataset associated with a blood or a blood derivative (e.g., plasma) sample, wherein the dataset comprises quantitative data for at least three, or at least four, or at least five, or all six protein markers selected from the group consisting of apolipoprotein H, CD40, haptoglobin, interleukin-8 (IL-8), monocyte chemoattractant protein-1 (MCP-1) and tumor necrosis factor receptor II (TNF-RII). The test sample dataset is then compared to a reference dataset containing quantitative data from the identical group of protein markers obtained from one or more reference samples used to generate the predictive model. In one embodiment, both the test dataset and the reference dataset(s) used to generate the predictive model comprise quantitative data for at least the three plasma markers apolipoprotein H, MCP-1 and TNF-RII. In another embodiment, the quantitative data is a measurement of protein concentration.

In another embodiment, a predictive model is used in classifying a test sample obtained from a mammalian subject by obtaining a dataset associated with a blood or blood derivative (e.g., plasma) sample, wherein the dataset comprises quantitative data for at least three, or at least four, or at least five, or at least six, or at least seven, or all eight protein markers selected from the group consisting of apolipoprotein CIII, CD40, haptoglobin, granulocyte-macrophage colony stimulating factor (GM-CSF), immunoglobulin A (IgA), macrophage inflammatory protein 1 alpha (MIP-1 ), tissue factor and tumor necrosis factor-alpha (TNF- ). As described in the embodiment above, the test sample dataset is compared to an identical dataset *(i.e.,* a reference dataset comprised of quantitative data from the identical group of protein biomarkers) obtained from one or more reference samples used to generate the predictive model. This method will classify the sample as being derived from a healthy individual or an individual with COPD, in particular slowly declining COPD. In a further embodiment, both the test dataset and the reference dataset(s) used to generate the predictive model comprise quantitative data for at least the three plasma markers IgA, MIP-1 and tissue factor. In another embodiment, the quantitative data is a measurement of protein concentration.

In a further embodiment, a predictive model is used in classifying a test sample obtained from a mammalian subject as being derived from an individual with a certain severity of COPD, particularly rapidly declining COPD or slowly declining COPD. A first step in said method is obtaining a dataset associated with a blood or blood derivative (e.g., plasma) sample, wherein the dataset comprises quantitative data for at least three, or at least four, or at least five, or at least six, or at least seven, or at least eight, or all nine protein markers selected from the group consisting of alpha-1 antitrypsin, C-reactive protein (CRP), fibrinogen, granulocyte-macrophage colony stimulating factor (GM-CSF), interleukin-4 (IL-4), macrophage-derived chemokine (MDC), tissue factor, tumor necrosis factor receptor II (TNFRII) and soluble vascular cell adhesion molecule 1 (sVCAM-1). The test sample dataset is compared to an identical dataset *(i.e.,* a reference dataset comprised of quantitative data from the identical group of protein biomarkers) obtained from one or more reference samples used to generate the predictive model. In a further embodiment, the dataset comprises quantitative data for at least the three plasma markers MDC, tissue factor and sVCAM-1. In another embodiment, the quantitative data is a measurement of protein concentration.

As used herein, individuals with rapid declining COPD lose on average 40 ml FEV1 (forced expiratory volume in 1 second) or more per year as measured by spirometry. Individuals with slowly declining COPD lose on average less than 40 ml FEV1 per year.

Reference to open-ended terms such as "comprises" allows for additional elements or steps. Occasionally phrases such as "one or more" are used with or without open-ended terms to highlight the possibility of additional elements or steps.

Unless explicitly stated reference to terms such as "a" or "an" is not limited to one. For example, "a cell" does not exclude "cells." Occasionally phrases such as one or more are used to highlight the possible presence of a plurality.

Other features and advantages of the present invention are apparent from the additional descriptions provided herein including the different examples. The provided examples illustrate different components and methodology useful in practicing the present invention. The examples do not limit the claimed invention. Based on the present disclosure the skilled artisan can identify and employ other components and methodology useful for practicing the present invention.

### BRIEF DESCRIPTION OF THE DRAWINGS

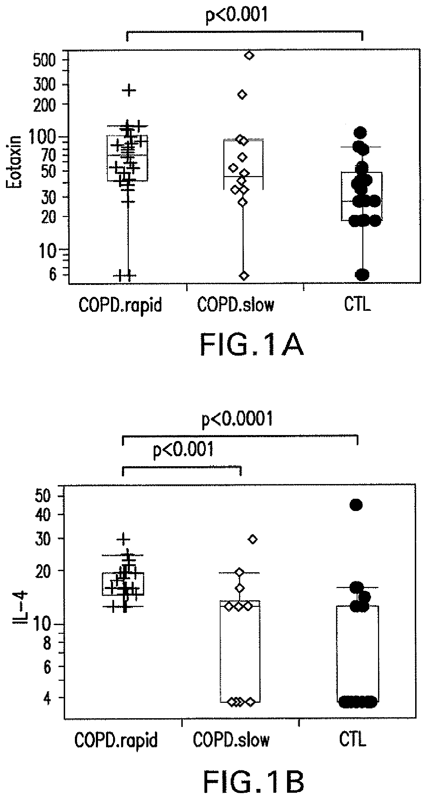

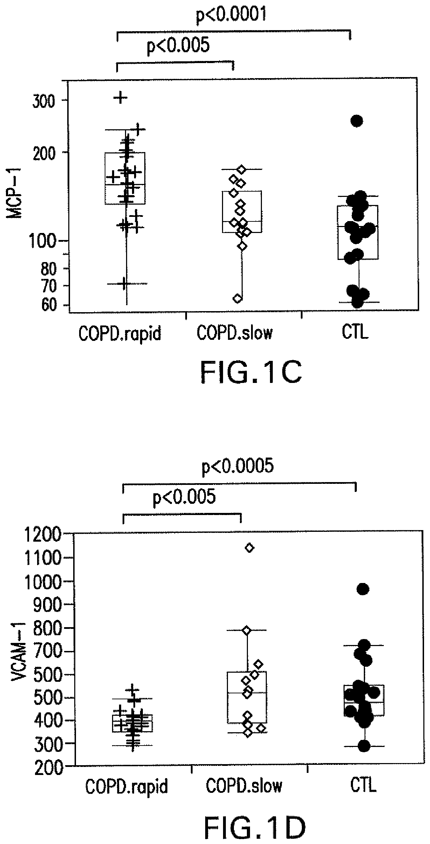

Figure 1 illustrates four examples of plasma markers, (A) eotaxin, (B) IL-4, (C) MCP-1 and (D) sVCAM-1, present at significantly different levels between COPD rapid decliners ("+") and COPD slow decliners (" ") and/or healthy subjects (" ") as determined by univariate analysis.

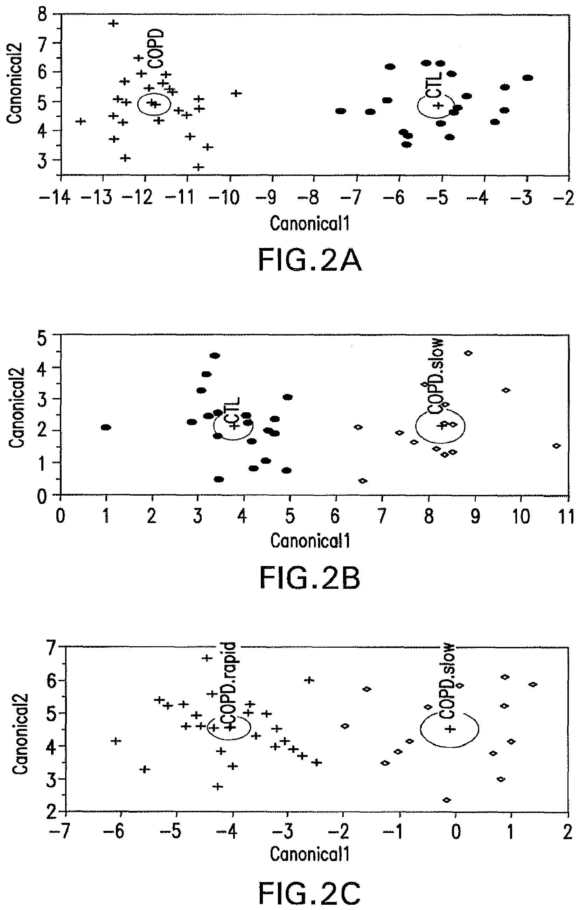

Figure 2 illustrates a graphical output from a linear discriminant analysis (LDA) using different signatures, (A) COPD rapid decliners and healthy controls, (B) COPD slow decliners and healthy controls, and (C) COPD rapid and slow decliners (" ", control (CTL); "+", COPD or COPD rapid; " ", COPD slow). LDA analysis measures the distance from each point in the data set to each group's multivariate mean and classifies the point to the closest group. The distance measure used is the Mahalanobis distance, which takes into account the variance and covariance between the variables. Each multivariate mean is a labeled circle. The size of the circle corresponds to a 95% confidence limit for the mean. Groups that are significantly different tend to have non-intersecting circles. These graphs were generated using JMP software, v5.0.1 from SAS Institute (Cary, NC).

### DETAILED DESCRIPTION OF THE INVENTION

Provided herein is the disclosure of both molecular factors and definitive tests to positively identify patients with COPD and/or differentiate between affected patients that may experience rapid lung decline or slow lung decline as a result of COPD. Because COPD is a disease that slowly progresses over decades with significant limitations in lung function appearing only at a late stage of the disease, early detection of biomarkers capable of 1) helping with the diagnosis of COPD, 2) predicting the rate of lung function decline, and 3) monitoring drug efficacy in a timely manner in clinical trials would be very useful.

### I. Plasma Biomarkers

Disclosed is the identification of plasma proteins which levels are statistically different in patients with COPD compared to healthy controls (see Example 3). Out of 89 plasma markers evaluated and analyzed by univariate analysis as described herein, 25 markers are statistically different between COPD rapid decliners and healthy controls (p<0.05; see Table *3, infra*)*,* 4 markers are statistically different between COPD slow decliners and healthy controls (p< 0.05; see Table 4, *infra*)*,* and 10 markers are statistically different between COPD rapid and slow decliners (p<0.05; see Table 5, *infra*)*.* Since none of these plasma markers are capable, on their own, to clearly distinguish COPD patients from controls, multivariate analysis of the data using the Linear Discriminant Analysis (LDA) method was performed. This method identified three groups of plasma biomarkers capable of accurately distinguishing between COPD rapid decliners and healthy controls (Signature 1), COPD slow decliners and healthy controls (Signature 2), and COPD rapid and slow decliners (Signature 3).

COPD biomarkers detectable in plasma are preferable since blood is easily obtained and more reliable than lung secretions contained in bronchoalveolar lavage or induced sputum. Interestingly, there is growing evidence that both local and systemic inflammation takes place in COPD (reviewed by Wouters, 2005, Proc. Am. Thorac. Soc. 2:26-33). It is yet unclear, however, whether COPD results from a general defect in the regulation of inflammation that manifests in the lung irritated by smoke or an uncontrolled inflammatory process that specifically takes place in the lung with systemic overflow. In either case, peripheral blood represents a convenient and rich source of information on COPD progression.

Signature 1 is a multi-analyte panel composed of the following 6 protein markers: apolipoprotein H, CD40, haptoglobin, interleukin-8 (IL-8), monocyte chemoattractant protein-1 (MCP-1) and tumor necrosis factor receptor II (TNF-RII). Signature 1 can correctly identify a plasma sample from a COPD rapid decliner in 93% of the cases (sensitivity) and a plasma sample from a healthy subject in 86% of the cases (specificity), for an overall accuracy in distinguishing between the two groups of 90% (see Table 6 in Example 3, *infra*)*.*

Signature 2 is a multi-analyte panel composed of the following 8 protein markers: apolipoprotein CIII, CD40, granulocyte-macrophage colony stimulating factor (GM-CSF), haptoglobin, immunoglobulin A (IgA), macrophage inflammatory protein 1 alpha (MIP-1 ), tissue factor and tumor necrosis factor-alpha (TNF- ) (see Table 6 in Example 3, *infra*)*.* Signature 2 can correctly identify a plasma sample from a COPD slow decliner (versus a healthy subject) with a 91% sensitivity and a 96% specificity, for an overall accuracy of 94%.

Signature 3 is a multi-analyte panel composed of the following 9 protein markers: alpha-1 antitrypsin, C-reactive protein (CRP), fibrinogen, granulocyte-macrophage colony stimulating factor (GM-CSF), interleukin-4 (IL-4), macrophage-derived chemokine (MDC), tissue factor, tumor necrosis factor receptor II (TNF-RII) and vascular cell adhesion molecule 1 (sVCAM-1). Signature 3 can correctly identify that a plasma sample is from a COPD rapid decliner in 95% of the cases (sensitivity) and that a plasma sample is from a COPD slow decliner in 86% of the cases (specificity), for an overall accuracy of distinguishing between the two groups of 92% (see Table 6 in Example 3, *infra*)*.*

Table 1 provides further information about the proteins identified in Signatures 1-3 disclosed herein. The listed accession numbers correspond to entries within the National Center for Biotechnology Information (NCBI) database maintained by the National Institutes of Health.

**Table 1:**

| **Protein** | **Other names** | **Human polynucleotide accession no. (NCBI)** | **Human protein accession no. (NCBI)** |
|---|---|---|---|
| Apolipoprotein H | Beta-2 glycoprotein I | NM_000042 | NP_000033 |
| CD40 | CD40L receptor | NM_001250 | NP_001241 |
| Haptoglobin | Hp2-alpha | NM_005143 | NP_005134 |
| IL-8 | Interleukin-8 | NM_000584 | NP_000575 |
| MCP-1 | CCL2 | NM_002982 | NP_002973 |
| TNF-RII | TNFRSF1B | NM_001066 | NP_001057 |
| Apolipoprotein CIII | Apoc3 | NM_000040 | NP_000031 |
| GM-CSF | Colony stimulating factor 2 | NM_000758 | NP_000749 |
| IgA | Immunoglobulin type A | BC087841 | AAH87841 |
| MIP-1 | CCL3 | NM_002983 | NP_002974 |
| Tissue factor | Coagulation factor III | NM_001993 | NP_001984 |
| TNF- | TNF superfamily member 2 | NM_000594 | NP_000585 |
| 1-antitrypsin | Serpin A1 | NM_000295 | NP_000286 |
| CRP | C-reactive protein | NM_000567 | NP_000558 |
| Fibrinogen | FGA | NM_000508 | NP_000499 |
| MDC | CCL22 | NM_002990 | NP_002981 |
| sVCAM-1 | Soluble VCAM-1 | NM_001078 | NP_001069 |
| IL-4 | Interleukin-4 | AF395008 | AAK71324 |

Proteins frequently exist in a sample in a plurality of different forms. These forms can result from either or both of pre- and post-translational modifications. Pre-translationally modified forms include allelic variants, splice variants and RNA editing forms. Post-translationally modified forms include forms resulting from proteolytic cleavage (e.g., cleavage of a signal sequence or fragments of a parent protein), glycosylation, phosphorylation, lipidation, oxidation, methylation, cysteinylation, sulphonation and acetylation.

Thus, in addition to the specific biomarker sequences identified in this application by name or accession number, the invention also contemplates the detection in a test sample of naturally occurring variants that are at least 90%, or at least 95%, or at least 97%, identical to the exemplified biomarker sequences (either nucleotide or protein sequences) listed in Table 1. Said biomarker variants shall have utility for the methods of the present invention and shall be detected via methods, as disclosed herein, used to detect the original biomarkers listed in column 1 of Table 1 (e.g., cross-reactivity with antibodies specific to the protein biomarkers listed in Table 1). These variants include but are not limited to polymorphisms, splice variants and mutations.

The term percent "identity," in the context of two or more nucleic acid or polypeptide sequences, refers to two or more sequences or subsequences that have a specified percentage of nucleotides or amino acid residues that are the same, when compared and aligned for maximum correspondence, as measured using a sequence comparison algorithm (e.g., BLASTP and BLASTN or other algorithms available to persons of skill) or by visual inspection. Depending on the application, the percent identity can exist over a region of the sequence being compared (e.g., over a functional domain) or, alternatively, exists over the full length of the two sequences to be compared. For sequence comparison, typically one sequence acts as a reference sequence to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence. Optimal alignment of sequences for comparison can be conducted, *e.g.,* by the local homology algorithm of Smith and Waterman (1981, Adv. Appl. Math. 2:482), by the homology alignment algorithm of Needleman and Wunsch (1970, J. Mol. Biol. 48:443), by the search for similarity method of Pearson and Lipman (1988, Proc. Natl. Acad. Sci. USA 85:2444), by computerized implementations of these algorithms (GAP, BESTFIT, FASTA, and TFASTA in the Wisconsin Genetics Software Package, Genetics Computer Group, 575 Science Dr., Madison, WI), or by visual inspection (see, generally, Ausubel, F M, et al., Current Protocols in Molecular Biology, 4, John Wiley & Sons, Inc., Brooklyn, N.Y., A.1E.1-A.1F.11, 1996-2004). One example of an algorithm that is suitable for determining percent sequence identity and sequence similarity is the BLAST algorithm, which is described in Altschul et al. (1990, J. Mol. Biol. 215:403-410). Software for performing BLAST analyses is publicly available through the National Center for Biotechnology Information (www.ncbi.nlm.nih.gov).

### II. Biomarker Detection

A protein biomarker is characterized by molecular weight and/or its known protein identity. Protein biomarkers can be resolved from other proteins in a sample by using a variety of fractionation techniques, *e.g*., chromatographic separation coupled with mass spectrometry, protein capture using immobilized antibodies and traditional immunoassays. Detection paradigms that can be employed to this end include optical methods, electrochemical methods (voltametry and amperometry techniques), atomic force microscopy, and radio frequency methods, *e.g.*, multipolar resonance spectroscopy. Illustrative of optical methods, in addition to microscopy, both confocal and non-confocal, are detection of fluorescence, luminescence, chemiluminescence, absorbance, reflectance, transmittance, and birefringence or refractive index (*e.g*., surface plasmon resonance, ellipsometry, a resonant mirror method, a grating coupler waveguide method and interferometry).

When detecting or measuring a protein in a sample (*e.g.,* a test sample as described herein), the ability to differentiate between both different proteins and different forms of the same protein depends upon the nature of the differences between the proteins and the method of detection used. For example, an immunoassay using a monoclonal antibody will detect all forms of a protein containing the eptiope and will not distinguish between them. However, a sandwich immunoassay that uses two antibodies directed against different epitopes on a protein will detect all forms of the protein that contain both epitopes and will not detect those forms that contain only one of the epitopes. When a particular form (or a subset of particular forms) of a protein is a better biomarker than the collection of different forms detected together by a particular method, the power of the assay may suffer. Thus, it may be useful to employ an assay method that distinguishes between forms of a protein and that specifically detects and measures a desired form or forms of the protein. Distinguishing different forms of a protein analyte or specifically detecting a particular form of a protein analyte is referred to as "resolving" the analyte.

In one embodiment of the invention, blood samples, or samples derived from blood (*e.g*. plasma, serum), are assayed for the presence of one or more of the protein markers disclosed as members of the multi-analyte biomarker signatures described herein. Typically, a blood sample is drawn, and a derivative product, such as plasma or serum, is tested. Such protein biomarkers may be detected through the use of specific binding members. For example, the use of antibodies for this purpose is of particular interest. Various formats find use for such assays, including the following: antibody arrays; enzyme-linked immunosorbent assays (ELISA) and radioimmunoassay (RIA) formats; binding of labeled antibodies in suspension/solution and detection by a method which includes, but is not limited to, flow cytometry and mass spectroscopy. Detection may utilize one or a panel of antibodies, preferably a panel of antibodies in an array format.

The biomarkers of the present invention can also be detected by mass spectrometry, a method that employs a mass spectrometer to detect gas phase ions. Examples of mass spectrometers are time-of-flight, magnetic sector, quadrupole filter, ion trap, ion cyclotron resonance, electrostatic sector analyzer and hybrids of these. The mass spectrometer can be a laser desorption/ionization (LDI) mass spectrometer. In laser desorption/ionization mass spectrometry, the protein analytes are placed on the surface of a mass spectrometry probe, a device adapted to engage a probe interface of the mass spectrometer and to present an analyte to ionizing energy for ionization and introduction into a mass spectrometer. A laser desorption mass spectrometer employs laser energy, typically from an ultraviolet laser, but also from an infrared laser, to desorb analytes from a surface, to volatilize and ionize them and make them available to the ion optics of the mass spectrometer. The analyis of proteins by LDI can take the form of MALDI or of SELDI (see U.S. Publication No. US20070172902 in the name of Zhang et al.).

Mass spectrometry is also a particularly powerful methodology to resolve different forms of a protein because the different forms typically have different masses that can be resolved by the technique. Accordingly, if one form of a protein is a superior biomarker for detection by the methods disclosed than another form of the biomarker, mass spectrometry may be able to specifically detect and measure the useful form where a traditional immunoassay fails both to distinguish the forms and to specifically detect the useful biomarker.

Mass spectrometry can also be combined with an immunoassay. First, a biospecific capture reagent (*e.g*., an antibody that recognizes the biomarker and forms of it) is used to capture the biomarker of interest. Preferably, the biospecific capture reagent is bound to a solid phase, such as a bead, a plate, a membrane or an array. After unbound materials are washed away, the captured analytes are detected and/or measured by mass spectrometry. Various forms of mass spectrometry are useful for detecting the protein forms, as described above, including laser desorption approaches, such as traditional MALDI or SELDI, and electrospray ionization.

A test sample can also be analyzed by means of a biochip. Biochips generally comprise solid substrates and have a generally planar surface to which a capture reagent (also called an adsorbent or affinity reagent) is attached. Frequently, the surface of a biochip comprises a plurality of addressable locations, each of which has the capture reagent bound there. Protein biochips are biochips adapted for the capture of polypeptides. Many protein biochips are described in the art. These include, for example, protein biochips produced by Ciphergen Biosystems, Inc. (Fremont, CA), Zyomyx (Hayward, CA), Invitrogen (Carlsbad, CA), Biacore (Uppsala, Sweden) and Procognia (Berkshire, UK). Examples of such protein biochips are described in the following patents or published patent applications: U.S. Patent No. 6,225,047 (Hutchens & Yip); U.S. Patent No. 6,537,749 (Kuimelis and Wagner); U.S. Patent No. 6,329,209 (Wagner et al.); PCT International Publication No. WO 00/56934 (Englert et al.); PCT International Publication No. WO 03/048768 (Boutell et al.); and, U.S. Patent No. 5,242,828 (Bergstrom et al.).

### III. Classifying Samples

The present invention discloses methods used for rapid detection and/or accurate diagnosis of chronic obstructive pulmonary disease (COPD) in a subject, classifying and/or identifying samples derived from a subject diagnosed with COPD according to the severity of the disease, and identifying and assessing the extent of COPD progression in a subject (disease monitoring/staging). Classifying and/or identifying test samples as being derived from healthy controls, rapidly declining COPD patients or slowly declining COPD patients can also be used to predict and/or monitor a response to a therapeutic regimen, including but not limited to monitoring drug efficacy during a clinical trial. Thus, the present invention includes methods of evaluating the efficacy of therapeutic agents and therapeutic regimens; disease staging and classification; and the like. Early detection can be used to determine the occurrence of developing COPD, thereby allowing for intervention with appropriate preventive or protective measures.

In methods of classifying and/or identifying a test sample as being obtained from a subject with COPD (either slowing declining or rapidly declining COPD), diagnosing a patient with COPD, or classifying the severity of COPD in a patient, the expression pattern in a test sample, including but not limited to blood, serum and plasma, of one or more of the protein markers provided herein is obtained and compared to control values to determine a diagnosis/classification. For example, a blood-derived sample (test sample) may be applied to a specific binding agent (e.g., antibody) or panel of specific binding agents to determine the presence of the markers of interest and/or quantify the markers within the sample. The analysis will generally include detecting and/or quantifying at least one of the markers described herein, e.g., apolipoprotein H, CD40, haptoglobin, interleukin-8 ("IL-8"), monocyte chemoattractant protein-1 ("MCP-1 "), tumor necrosis factor receptor II ("TNF-RII"), apolipoprotein CIII, granulocyte-macrophage colony stimulating factor ("GM-CSF"), immunoglobulin A ("IgA"), macrophage inflammatory protein 1 alpha ("MIP-1 "), tissue factor, tumor necrosis factor-alpha ("TNF- "), alpha-1 antitrypsin, C-reactive protein ("CRP"), fibrinogen, interleukin-4 (IL-4), macrophage-derived chemokine ("MDC"), and soluble vascular cell adhesion molecule 1 ("sVCAM-1"); usually at least two of the markers, more usually at least three of the markers, and may include 4, 5, 6, 7 or up to all of the markers, depending on the particular classification desired.

When classifying a test sample as derived from a subject with COPD and/or diagnosing a subject with COPD, in particular classifying between rapidly declining COPD and healthy controls, a preferred set of markers comprises at least three of the following multi-analyte panel: apolipoprotein H, CD40, haptoglobin, IL-8, MCP-1 and TNF-RII; and may include, 4, 5 or all 6 of them. This multi-analyte panel represents Signature 1 as described herein. The preferred at least three markers to quantify for this method are apoliprotein H, MCP-1 and TNF-RII, providing an overall accuracy of correctly identifying a plasma sample from a COPD rapid decliner versus a healthy subject of approximately 89% (see Table 6, *infra*)*.* The degree of accuracy is increased to approximately 90% by assessing quantitative data for all 6 of the listed biomarkers within Signature 1 (see Table 6, *infra*)*.*

When classifying a test sample as derived from a subject with COPD and/or diagnosing a subject with COPD, in particular classifying between slowly declining COPD and healthy controls, a preferred set of markers comprises at least three of the following multi-analyte panel: apolipoprotein CIII, CD40, haptoglobin, GM-CSF, IgA, MIP-1 , tissue factor and TNF- ; and may include 4, 5, 6, 7 or all 8 of them. This multi-analyte panel represents Signature 2 as described herein. The preferred at least three markers to quantify for this method are IgA, MIP-1 and tissue factor, providing an overall accuracy of correctly identifying a plasma sample from a COPD slow decliner versus a healthy subject of approximately 76% (see Table 6, *infra*)*.* The degree of accuracy is increased to approximately 78% by further quantifying GM-CSF (for a total of 4 biomarkers) and increased to approximately 83% by further quantifying GM-CSF and apolipoprotein CIII (for a total of 5 biomarkers). The degree of accuracy is increased to approximately 94% by assessing quantitative data for all 8 of the listed biomarkers (see Table 6, *infra*)*.*

When classifying a test sample as derived from a subject with COPD and/or diagnosing a subject with COPD, in particular classifying between rapidly and slowly declining COPD, a preferred set of markers comprises at least three of the following multi-analyte panel: alpha-1 antitrypsin, CRP, fibrinogen, GM-CSF, IL-4, MDC, tissue factor, TNF-RII and sVCAM-1; and may include 4, 5, 6, 7, 8 or all 9 of them. This multi-analyte panel represents Signature 3 as described herein. The preferred at least three markers to quantify for this method are MDC, tissue factor and sVCAM-1, providing an overall accuracy of correctly distinguishing a plasma sample derived from a COPD rapid decliner as opposed to a plasma sample derived from a COPD slow decliner of approximately 80% (see Table 6, *infra*)*.* The degree of accuracy is increased to approximately 85% by further quantifying IL-4 (for a total of 4 biomarkers), and the degree of accuracy is increased to approximately 92% by assessing quantitative data for all 9 of the listed biomarkers. When staging the severity of COPD *(i.e.,* rapid decliner versus slow decliner), an individual test dataset will be compared against one or more reference datasets obtained from disease samples of a known stage, constructing a model that predicts stage and inputting a dataset in that model to obtain a predicted staging.

Thus, using the information disclosed herein, the present invention provides for computer-implemented methods for classifying a test sample obtained from a mammalian subject, comprising (a) obtaining a dataset associated with said sample, wherein said obtained dataset *(i.e.,* test dataset) comprises quantitative data for at least three protein markers selected from a multi-analyte panel selected from the group consisting of: (i) apolipoprotein H, CD40, haptoglobin, interleukin-8 (IL-8), monocyte chemoattractant protein-1 (MCP-1) and tumor necrosis factor receptor II (TNF-RII); (ii) apolipoprotein CIII, CD40, granulocyte-macrophage colony stimulating factor (GM-CSF), interleukin-4 (IL-4), haptoglobin, immunoglobulin A (IgA), macrophage inflammatory protein 1 alpha (MIP-1 ), tissue factor and tumor necrosis factor-alpha (TNF- ); and, (iii) alpha-1 antitrypsin, C-reactive protein (CRP), fibrinogen, granulocyte-macrophage colony stimulating factor (GM-CSF), macrophage-derived chemokine (MDC), tissue factor, tumor necrosis factor receptor II (TNFRII) and soluble vascular cell adhesion molecule 1 (sVCAM-1); (b) inputting said obtained dataset into an analytical process on a computer that compares said obtained dataset against one or more reference datasets; and, (c) classifying said sample according to the output of said analytical process, wherein said classification is selected from the group consisting of a COPD classification and a healthy classification. In a further embodiment, a COPD classification represents either a rapidly declining COPD classification and/or a slowly declining COPD classification.

The present invention further provides for methods for classifying a test sample obtained from a human subject, comprising: (a) obtaining a dataset associated with said test sample, wherein said obtained dataset comprises quantitative data for at least three protein markers selected from a multi-analyte panel selected from the group consisting of: (i) apolipoprotein H, CD40, haptoglobin, interleukin-8 (IL-8), monocyte chemoattractant protein-1 (MCP-1) and tumor necrosis factor receptor II (TNF-RII); (ii) apolipoprotein CIII, CD40, granulocyte-macrophage colony stimulating factor (GM-CSF), haptoglobin, immunoglobulin A (IgA), macrophage inflammatory protein 1 alpha (MIP-1 ), tissue factor and tumor necrosis factor-alpha (TNF- ); and, (iii) alpha-1 antitrypsin, C-reactive protein (CRP), fibrinogen, granulocyte-macrophage colony stimulating factor (GM-CSF), interleukin-4 (IL-4), macrophage-derived chemokine (MDC), tissue factor, tumor necrosis factor receptor II (TNFRII) and soluble vascular cell adhesion molecule 1 (sVCAM-1); (b) transforming said obtained dataset to classify said test sample using an analytical process that compares said obtained dataset against one or more reference datasets; and, (c) classifying said test sample according to the output of said analytical process, wherein said classification is selected from the group consisting of a chronic obstructive pulmonary disease (COPD) classification and a healthy classification.

The classification methods described herein may be used to identify a subject *(e.g.,* a human patient) for diagnosis or disease staging purposes in order to accurately develop a course of treatment most suitable for said individual. The classification methods may also be used to help calculate and assess efficacy of a drug for treating COPD during a clinical trial. Thus, once the subject has been classified, this information can be transformed, for example, to generate a more effective treatment plan to limit further development of COPD in said subject or to determine efficacy of a drug candidate for use in COPD treatment.

The methods disclosed herein can be practiced with the determination of the concentration in a test plasma sample of at least a subset of the three biomarkers signatures described herein. In certain embodiments, greater than 90% accuracy in making a correct diagnosis is provided by the disclosed methods.

The methods described herein may be implemented using any device capable of implementing the methods. Examples of devices that may be used include, but are not limited to, electronic computational devices, including computers of all types. When the methods described herein are implemented on a computer, the computer program that may be used to configure the computer to carry out the steps of the methods may be contained in any computer readable medium capable of containing the computer program. Examples of computer readable medium that may be used include, but are not limited to, diskettes, CD-ROMs, DVDs, ROM, RAM, and other memory and computer storage devices. The computer program that may be used to configure the computer to carry out the steps of the methods may also be provided over an electronic network, for example, over the internet, world wide web, an intranet, or other network.

The methods described herein may be implemented in a system comprising a processor and a computer readable medium that includes program code means for causing the system to carry out the steps of the methods. The processor may be any processor capable of carrying out the operations needed for implementation of the methods. The program code means may be any code that when implemented in the system can cause the system to carry out the steps of the methods described herein. Examples of program code means include, but are not limited to, instructions to carry out the methods described in this application written in a high level computer language, such as C++, Java, or Fortran; instructions to carry out the methods described in this application written in a low level computer language, such as assembly language; or, instructions to carry out the methods described herein in a computer executable form, such as compiled and linked machine language.

### IV. Data Analysis

The quantitation of markers in a sample (e.g., a test sample or a reference sample) is determined by the methods described above and as known in the art. Quantitative data obtained from a test sample ("obtained dataset" or "test dataset," as used interchangeably herein) is subjected to an analytic classification process that compares the obtained dataset to one or more reference datasets. In one embodiment, the raw data, including but not limited to protein concentration data for the tested biomarkers, is quantified and compared to a predictive model generated by assessing one or more reference populations using the same quantitative data as gathered from the test sample. The predictive model is generated using a training set of data from reference populations, wherein both the training dataset and the test (or obtained) dataset is comprised of the same quantitative information. For example, in one embodiment of the present invention when performing a method for classifying a test sample or diagnosing a subject with rapid declining COPD, a dataset associated with a test sample consisting of circulating plasma concentration data of apolipoprotein H, MCP-1 and TNF-RII is compared to a predictive model generated using the same set of concentration data *(i.e.,* circulating plasma concentration of apolipoprotein H, MCP-1 and TNF-RII) obtained from both healthy individuals (reference sample 1) and rapidly declining COPD individuals (reference sample 2).

Thus, in preferred embodiments, the methods of the invention use a classifier for diagnosing or classifying COPD. The classifier can be based on any appropriate pattern recognition method that receives an input comprising a multi-analyte profile and provides an output comprising data indicating to which group a test sample belongs. The classifer can be trained with training data from one or more reference population(s) of subjects (reference dataset(s)). Typically, the training data comprise, for each of the subjects in the training population, a multi-analyte profile comprising quantitative measurements of biomarker proteins in a suitable sample taken from the patient.

An analytic classification process may use any one of a variety of statistical analytic methods to manipulate the quantitative data and provide for classification of the test sample. Examples of useful methods include linear discriminant analysis (LDA), recursive feature elimination, a prediction analysis of microarray, a logistic regression, a CART algorithm, a FlexTree algorithm, a LART algorithm, a random forest algorithm, a MART algorithm and machine learning algorithms. A preferred analytic method is LDA. Using any one of these methods, one or more reference datasets is used to generate a predictive model. In the generation of such a model, a dataset comprising control and/or diseased samples is used as a training set. A training set will contain data for the same group of the markers quantified in the test sample. Examples of predictive models used to distinguish between rapidly declining COPD patients and healthy subjects, slowly declining COPD patients and healthy subjects, and rapidly declining and slowly declining COPD patients are provided herein, for example see Example 3.

The predictive models demonstrated herein utilize the results of multiple protein level determinations of the biomarkers contained within Signatures 1, 2 or 3 described herein, or a subset of biomarkers within said signatures, and provide a mechanism that will classify, with a desired degree of accuracy, an individual as belonging to a particular state, wherein a state may be rapidly declining COPD, slowly declining COPD or healthy (no disease state). Thus, classification of interest includes, without limitation, the assignment of a test sample to one or more of the following states: i) a COPD state, including a rapidly declining COPD state or a slowly declining COPD state; and, ii) a healthy state (no disease state).

Classifications also may be made by determining whether a comparison between an obtained dataset and a reference dataset yields a statistically significant difference. If so, then the sample from which the dataset was obtained is classified as not belonging to the reference dataset class. Conversely, if such a comparison is not statistically significant from the reference dataset, then the sample from which the dataset was obtained is classified as belonging to the reference dataset class.

Classification can be made according to predictive modeling methods that set a threshold for determining the probability that a sample belongs to a given class. The probability of the methods of the present invention is at least about 70%, preferably at least about 80%, and more preferably at least about 90% or higher. The predictive ability of a model may be evaluated according to its ability to provide a quality metric, *e.g*. accuracy, of a particular value or range of values. In one embodiment, a desired quality threshold has the ability to classify a test sample with an accuracy of at least about 70%, at least about 75%, at least about 80%, at least about 85%, or at least about 90% or higher. The term "accuracy" refers to the computed ability of an individual marker or a combination of markers to correctly identify a disease state (*e.g.,* COPD) from a control state (*e.g*., healthy). As is known in the art, the relative sensitivity and specificity of a predictive model can be tuned to favor either the selectivity metric or the sensitivity metric, where the two metrics have an inverse relationship. The term "sensitivity" refers to the ability of an individual marker or a combination of markers to correctly identify a disease state, while the term "specificity" refers to the ability of the marker(s) to correctly identify a normal (*e.g*., non-diseased) state. The limits in a model as described above can be adjusted to provide a selected sensitivity or specificity level, depending on the particular requirements of the test being performed. One or both of sensitivity and specificity may be at least about at least about 60%, at least about 65%, at least about 70%, at least about 75%, at least about 80%, at least about 85%, or at least about 90% or higher.

The raw data may be initially analyzed by measuring the values (*e.g*., concentration) for each marker, usually in triplicate or in multiple triplicates. The data may be manipulated, for example, raw data may be transformed using standard curves, and the average of triplicate measurements used to calculate the average and standard deviation for each patient. These values may be transformed before being used in the models, *e.g.* log-transformed, Box-Cox transformed (see Box and Cox (1964) J. Royal Stat. Soc., Series B, 26:211-246). The data are then input into a predictive model, which will classify the sample according to the state. The resulting information may be transmitted to a patient, health professional or clinical research analyst.

To generate a predictive model for COPD states, a robust data set, comprising known control samples and/or samples corresponding to a classification of interest are used in a training set ("reference dataset"). A sample size is selected using generally accepted criteria. As discussed above, different statistical methods can be used to obtain a highly accurate predictive model. An example of such analysis using linear disciminant analysis (LDA) is provided in Example 3.

Linear discriminant analysis (LDA) attempts to classify a test sample or subject into one of two categories based on certain object properties. In other words, LDA tests whether object attributes measured in an experiment predict categorization of the objects. LDA typically requires continuous independent variables and a dichotomous categorical dependent variable. In the present invention, the quantitative values (*e.g*., protein concentration data) for a selected set of biomarkers disclosed herein across a subset of the training population serve as the requisite continuous independent variables. The clinical group classification of each member of the training population serves as the dichotomous categorical dependent variable.

LDA seeks the linear combination of variables that maximizes the ratio of between-group variance and within-group variance by using the grouping information. Implicitly, the linear weights used by LDA depend on how the quantitative value (*e.g*., protein concentration) of a biomarker across the training set separates in the two groups (*e.g.,* a COPD control group and a healthy control group). In some embodiments, LDA is applied to the data matrix of the N members in the training sample by K biomarkers in a combination of biomarkers described in the present invention. Then, the linear discriminant of each member of the training population is plotted. Ideally, those members of the training population representing a first control subgroup (*e.g*. healthy group) will cluster into one range of linear discriminant values (*e.g*., negative), and those members of the training population representing a second control subgroup (*e.g*. those subjects with COPD) will cluster into a second range of linear discriminant values (*e.g*., positive). The LDA is considered more successful when the separation between the clusters of discriminant values is larger. For more information on linear discriminant analysis, see Duda, Pattern Classification, Second Edition, 2001, John Wiley & Sons, Inc; and Hastie, 2001, The Elements of Statistical Learning, Springer, New York; Venables & Ripley, 1997, Modern Applied Statistics with s-plus, Springer, New York.

Quadratic discriminant analysis (QDA) takes the same input parameters and returns the same results as LDA. QDA uses quadratic equations, rather than linear equations, to produce results. LDA and QDA are interchangeable, and which to use is a matter of preference and/or availability of software to support the analysis. Logistic regression takes the same input parameters and returns the same results as LDA and QDA.

In one embodiment, hierarchical clustering is performed in the derivation of a predictive model, where the Pearson correlation is employed as the clustering metric. One approach is to consider a COPD dataset as a "learning sample" in a problem of "supervised learning." CART is a standard in applications to medicine (Singer (1999) Recursive Partitioning in the Health Sciences, Springer), which may be modified by transforming any qualitative features to quantitative features; sorting them by attained significance levels, evaluated by sample reuse methods for Hotelling's T² statistic; and suitable application of the lasso method. Problems in prediction are turned into problems in regression without losing sight of prediction, indeed by making suitable use of the Gini criterion for classification in evaluating the quality of regressions.

This approach has led to what is termed FlexTree (Huang et al., 2004, Proc. Natl. Acad. Sci. USA 101:10529-10534). FlexTree has performed very well in simulations and when applied to SNP and other forms of data. Software automating FlexTree has been developed. Recent efforts have led to the development of such an approach, termed LARTree or simply LART (Turnbull (2005) Classification Trees with Subset Analysis Selection by the Lasso, Stanford University). The name reflects binary trees, as in CART and FlexTree; the lasso, as has been noted; and the implementation of the lasso through what is termed LARS by Efron et al. (2004) Annals of Statistics 32:407-451. See, also, Huang et al., 2004, *supra.*

Other methods of analysis that may be used include logic regression (see, *e.g.,* Ruczinski et al., 2003, J. Comput. Graph. Stat. 12:475-512). Logic regression resembles CART in that its classifier can be displayed as a binary tree. It is different in that each node has Boolean statements about features that are more general than the simple "and" statements produced by CART.

Another approach is that of nearest shrunken centroids (Tibshirani et al., 2002, Proc. Natl. Acad. Sci. USA 99:6567-72). The technology is k-means-like, but has the advantage that by shrinking cluster centers, one automatically selects features (as in the lasso) so as to focus attention on small numbers of those that are informative. The approach is available as PAM software and is widely used. Two further sets of algorithms are random forests (Breiman et al., 2001, Mach. Learn. 45:5-32) and MART (Hastie et al., 2001, The Elements of Statistical Learning, Springer). These two methods are already "committee methods." Thus, they involve predictors that "vote" on outcome.

To provide significance ordering, the false discovery rate (FDR) may be determined. First, a set of null distributions of dissimilarity values is generated. In one embodiment, the values of observed profiles are permuted to create a sequence of distributions of correlation coefficients obtained out of chance, thereby creating an appropriate set of null distributions of correlation coefficients (see Tusher et al., 2001, Proc. Natl. Acad. Sci. USA 98, 5116-21; herein incorporated by reference). The set of null distribution is obtained by: permuting the values of each profile for all available profiles; calculating the pair-wise correlation coefficients for all profile; calculating the probability density function of the correlation coefficients for this permutation; and, repeating the procedure for N times, where N is a large number, usually about 300. Using the N distributions, one calculates an appropriate measure (mean, median, etc.) of the count of correlation coefficient values that their values exceed the value (of similarity) that is obtained from the distribution of experimentally observed similarity values at given significance level.

The FDR is the ratio of the number of the expected falsely significant correlations (estimated from the correlations greater than this selected Pearson correlation in the set of randomized data) to the number of correlations greater than this selected Pearson correlation in the empirical data (significant correlations). This cut-off correlation value may be applied to the correlations between experimental profiles.

Using the aforementioned distribution, a level of confidence is chosen for significance. This is used to determine the lowest value of the correlation coefficient that exceeds the result that would have been obtained by chance. Using this method, one obtains thresholds for positive correlation, negative correlation or both. Using this threshold(s), the user can filter the observed values of the pairwise correlation coefficients and eliminate those that do not exceed the threshold(s). Furthermore, an estimate of the false positive rate can be obtained for a given threshold. For each of the individual "random correlation" distributions, one can find how many observations fall outside the threshold range. This procedure provides a sequence of counts. The mean and the standard deviation of the sequence provide the average number of potential false positives and its standard deviation.

In an alternative analytical approach, variables chosen in the cross-sectional analysis are separately employed as predictors. Given the specific COPD outcome, the random lengths of time each patient will be observed, and selection of proteomic and other features, a parametric approach to analyzing survival may be better than the widely applied semi-parametric Cox model. A Weibull parametric fit of survival permits the hazard rate to be monotonically increasing, decreasing, or constant, and also has a proportional hazards representation (as does the Cox model) and an accelerated failure-time representation. All the standard tools available in obtaining approximate maximum likelihood estimators of regression coefficients and functions of them are available with this model.

In addition, the Cox models may be used, especially since reductions of numbers of covariates to manageable size with the lasso will significantly simplify the analysis, allowing the possibility of an entirely nonparametric approach to survival. These statistical tools are applicable to all manner of proteomic data. A set of biomarkers that can be easily determined, and that is highly informative regarding detection of individuals with clinically significant COPD, is provided.

In the development of a predictive model, it may be desirable to select a subset of markers, *i.e.* at least 3, at least 4, at least 5, at least 6, or up to the complete set of markers. Usually a subset of markers will be chosen that provides for the needs of the quantitative sample analysis (*e.g*., availability of reagents, convenience of quantitation, etc.) while maintaining a highly accurate predictive model. The selection of a number of informative markers for building classification models requires the definition of a performance metric and a user-defined threshold for producing a model with useful predictive ability based on this metric. For example, the performance metric may be the sensitivity and/or specificity of the prediction, as well as the overall accuracy of the prediction model. As described in Example 3, LDA was used in a training model to identify biomarkers relevant to COPD and certain stages of the disease. The selection of a subset of markers may be used for a forward selection or a backward selection of a marker subset. A number of markers may be selected that will optimize the performance of a model without the use of all the markers (see Table 6 in Example 3, *infra*)*.* One way to define the optimum number of terms is to choose the number of terms that produce a model with desired predictive ability (*e.g*., an accuracy of greater than 80%, or equivalent measures of sensitivity/specificity) that lies no more than one standard error from the maximum value obtained for this metric using any combination and number of terms used for the given analytic process.

### V. Reagents and Kits

Also provided are reagents and kits thereof for practicing one or more of the above-described methods. The subject reagents and kits thereof may vary greatly. Reagents of interest include reagents specifically designed for use in production of the above described expression profiles of circulating protein markers associated with COPD.

Thus, the invention provides kits for practicing one or more of the above-described methods that include at least one reagent specific for a COPD biomarker described herein, wherein the COPD biomarker is selected from the group consisting of apolipoprotein H, CD40, haptoglobin, interleukin-8 ("IL-8"), monocyte chemoattractant protein-1 ("MCP-1"), tumor necrosis factor receptor II ("TNF-RII"), apolipoprotein CIII, granulocyte-macrophage colony stimulating factor ("GM-CSF"), immunoglobulin A ("IgA"), macrophage inflammatory protein 1-alpha ("MIP-1 "), tissue factor, tumor necrosis factor-alpha ("TNF- "), alpha-1 antitrypsin, C-reactive protein ("CRP"), fibrinogen, interleukin-4 ("IL-4"), macrophage-derived chemokine ("MDC"), and soluble vascular cell adhesion molecule 1 ("sVCAM-1"). The expression of the one or more biomakers can be determined using said reagent that detects the one or more biomarkers. The kit is used to classify a test sample obtained from a human subject within a COPD group or a healthy group.

In one embodiment, said kit comprises reagents for detecting at least three protein markers selected from one or more of the biomarkers signatures described herein. Thus, said kit may comprise reagents for detecting at least three protein markers selected from a multi-analyte panel selected from the group consisting of:
(a) apolipoprotein H, CD40, haptoglobin, interleukin-8 (IL-8), monocyte chemoattractant protein-1 (MCP-1) and tumor necrosis factor receptor II (TNF-RII);
(b) apolipoprotein CIII, CD40, granulocyte-macrophage colony stimulating factor (GM-CSF), haptoglobin, immunoglobulin A (IgA), macrophage inflammatory protein 1alpha (MIP-1 ), tissue factor and tumor necrosis factor-alpha (TNF- ); and,
(c) alpha-1 antitrypsin, C-reactive protein (CRP), fibrinogen, granulocyte-macrophage colony stimulating factor (GM-CSF), interleukin-4 (IL-4), macrophage-derived chemokine (MDC), tissue factor, tumor necrosis factor receptor II (TNFRII) and soluble vascular cell adhesion molecule 1 (sVCAM-1).

A kit of the present invention may include reagents that are labeled compounds or agents useful to detect a polypeptide or an mRNA encoding a polypeptide corresponding to a COPD biomarker disclosed herein in a biological sample and means for determining the amount of the polypeptide or mRNA in the sample (e.g., an antibody that binds the polypeptide or an oligonucleotide probe that binds to DNA or mRNA encoding the polypeptide). One type of such reagent suitable for binding with a polypeptide corresponding to a COPD biomarker is an antibody or fragment thereof (including an antibody derivative) that binds to a marker of interest. Additionally, suitable reagents for binding with a nucleic acid (e.g., genomic DNA, mRNA, spliced mRNA, cDNA) include complementary nucleic acids. A variety of different array formats are known in the art, with a wide variety of different probe structures, substrate compositions and attachment technologies. In further embodiments, the reagent is directly or indirectly labeled with a detectable substance.

In certain embodiments, the expression of the one or more biomarkers is detected by: (a) detecting the expression of a polypeptide which is regulated by the one or more biomarkers; (b) detecting the expression of a polypeptide which regulates the biomarker; or, (c) detecting the expression of a metabolite of the biomarker.

For antibody-based kits, the kit can comprise, for example: (1) a first antibody (e.g., attached to a solid support) which binds a polypeptide corresponding to a biomarker of the invention; and, optionally (2) a second, different antibody that binds to either the polypeptide or the first antibody and is conjugated to a detectable label.

For oligonucleotide-based kits, the kit can comprise, for example: (1) an oligonucleotide, e.g., a detectably labeled oligonucleotide, which hybridizes to a nucleic acid sequence encoding a polypeptide corresponding to a biomarker of the invention, or (2) a pair of primers useful for amplifying a nucleic acid molecule corresponding to a biomarker of the invention.

The kit can also comprise other components, such as a buffering agent, a preservative, a protein stabilizing agent, and/or components necessary for detecting the detectable label. The kit may include reagents employed in the various methods, such as devices for withdrawing and handling blood samples, second stage antibodies, ELISA reagents; tubes, spin columns, and the like. Each component of the kit can be enclosed within an individual container and all of the various containers can be within a single package.

Representative array or kit compositions of interest include or consist of reagents for quantitation of at least three, at least four, at least five or all six markers selected from the group consisting of apolipoprotein H, CD40, haptoglobin, IL-8, MCP-1 and TNF-RII. This kit can be used to classify a test sample as and/or diagnosing a subject with rapidly declining COPD, as opposed to a healthy patient. The preferred at least three markers to quantify using reagents included within the kit may comprise or consist of apoliprotein H, MCP-1 and TNF-RII.

In another embodiment, a representative array or kit includes or consists of reagents for quantitation of at least three, at least four, at least five, at least six, at least seven or all eight markers selected from the group consisting of apolipoprotein CIII, CD40, haptoglobin, GM-CSF, IgA, MIP-1 , tissue factor and TNF- . This kit can be used to classify a test sample as and/or diagnosing a subject with slowly declining COPD, as opposed to a healthy patient. The preferred at least three markers to quantify using reagents included within the kit may comprise or consist of IgA, MIP-1 and tissue factor.

In yet a further embodiment, a representative array or kit includes or consists of reagents for quantitation of at least three, at least four, at least five, at least six, at least seven, at least eight or all nine markers selected from the group consisting of alpha-1 antitrypsin, CRP, fibrinogen, GM-CSF, IL-4, MDC, tissue factor, TNF-RII and sVCAM-1. This kit can be used to classify a test sample as and/or diagnosing a subject with rapid declining COPD, as opposed to slowly declining COPD. The preferred at least three markers to quantify using reagents included within the kit may comprise or consist of MDC, tissue factor and sVCAM-1.

The kits may further include a software package for statistical analysis of one or more phenotypes, and may include a reference database(s) for calculating the probability of classification within a predictive model.

In addition to the above components, the subject kits may further include instructions for practicing the subject methods and for interpreting the results of the assays performed using the kit. These instructions may be present in the subject kits in a variety of forms, one or more of which may be present in the kit. One form in which these instructions may be present is as printed information on a suitable medium or substrate, e.g., a piece or pieces of paper on which the information is printed, in the packaging of the kit, in a package insert, etc. Yet another means would be a computer readable medium, e.g., diskette, CD, etc., on which the information has been recorded. Yet another means that may be present is a website address which may be used via the internet to access the information at a removed site. Any convenient means may be present in the kits.

Examples are provided below further illustrating different features of the present invention. The examples also illustrate useful methodology for practicing the invention. These examples do not limit the claimed invention.

### EXAMPLE 1

### Collection of Study Subjects

Blood was collected in citrate buffer at the end of a 15-year longitudinal lung function study from 40 COPD patients and 20 healthy controls. COPD patients and healthy controls were either current or ex-smokers. The COPD patients were initially recruited in 1987-88 as part of the NIH funded National Lung Health Study, a 5-year longitudinal study of pulmonary function (Owens, 1991, Am. J. Med. 91:375-40S). Patients recruited were 35-59 years old current smokers with FEV1 ranging between 50% and 90% of predicted, an FEV1/forced vital capacity (FVC) less than 0.7 and presenting the characteristic respiratory symptoms of COPD. Spirometry was performed every year during the Lung Health Study. For the COPD patients evaluated in this study, additional spirometry was done at various intervals during the following 10 years at the University of Utah. Based on the 15-years longitudinal information on lung function, COPD patients were distributed in two distinct groups. The COPD sub-group of 26 rapid decliners is defined by those who lost 40 ml or more of FEV1 on average per year over the last 15 years. This is double or more the normal decline of 20 ml FEV1 reported for healthy smokers and non-smokers (Rennard, 1998, *supra*)*.* The COPD sub-group of 14 slow decliners is defined by those with an annual loss of FEV1 on average of less than 40 ml. At the time of blood collection, rapid decliners collectively had 64.0% of the predicted normal FEV1 after losing on average 20.2% FEV1 over the last 15 years. Slow decliners collectively had 72.3% of the predicted normal FEV1 after losing on average only 2.4% FEV1 over the last 15 years. All COPD patients had no other major illness, were not using corticosteroids throughout the study, and were of similar age and gender distribution. Healthy smoker/exsmoker controls were recruited throughout the study and were matched by sex, age and ethnicity. The clinical characteristics of the COPD patients and controls in the study are reported in Table 2.

**Table 2:**

| Group | Sex | Average age | Current % FEV1 | In the last 15 years | |
|---|---|---|---|---|---|
| | | | | % FEV1 loss | ml FEV1/year lost |
| Control smokers (n = 20) | 11 M; 9F | 56 | 99.4 | na | na |
| COPD Slow Decliners (n = 14) | 9M; 5F | 61 | 72.3 | < 12% (average: 2.4) | <40 ml (average: 24.0) |
| COPD Rapid Decliners (n = 26) | 13M; 13F | 62 | 64.0 | 12% (average: 20.2) | 40 ml (average: 62.3) |

| | | | | | |
|---|---|---|---|---|---|
| na = not applicable | | | | | |

### EXAMPLE 2

### Analysis of Plasma Markers

Frozen plasma samples were sent to Rules-Based Medicine, Inc. (Austin, TX) for analysis of markers in their proprietary Human Antigen MAP platform. Frozen plasma samples were thawed at room temperature, vortexed, spun at 13,000 x g for 5 minutes for clarification, and 40 uL was removed for markers analysis into a master microtiter plate. Using automated pipetting, an aliquot of each sample was introduced into one of the capture microsphere multiplexes of the Human Antigen MAP. These mixtures of sample and capture microspheres were thoroughly mixed and incubated at room temperature for 1 hour. Multiplexed cocktails of biotinylated, reporter antibodies for each multiplex were then added robotically and, after thorough mixing, were incubated for an additional hour at room temperature. Multiplexes were developed using an excess of streptavidin-phycoerythrin solution which was thoroughly mixed into each multiplex and incubated for 1 hour at room temperature. The volume of each multiplexed reaction was reduced by vacuum filtration and the volume increased by dilution into matrix buffer for analysis. Analysis was performed in a Luminex 100 instrument and the resulting data stream was interpreted using proprietary data analysis software developed at Rules-Based Medicine. For each multiplex, both calibrators and controls were included on each microtiter plate. Eight (8)-point calibrators were run in the first and last column of each plate and 3-level controls were included in duplicate. Testing results were determined first for the high, medium and low controls for each multiplex to ensure proper assay performance. Unknown values for each of the analytes localized in a specific multiplex were determined using 4 and 5 parameter, weighted and non-weighted curve fitting algorithms included in the data analysis package.

### EXAMPLE 3

### Data Analysis

Univariate analysis of individual markers - The significance of each marker between COPD rapid decliners versus healthy controls, COPD slow decliners versus healthy controls, and COPD rapid versus COPD slow decliners was assessed using Wilcoxon rank sum test. Exact p-values were determined using the Shift algorithm (Streitberg and Rohmel (1986) Exact Distribution for Permutations and Rank Test: An Introduction to Some Recently Published Algorithms. Statistical Software Newsletter. 12:10-17). In addition, the strength of the significance of each marker was further assessed using the more stringent false discovery rate. False Positive Rate (FPR), estimated by p-value, is the proportion of false positives among all the markers that in reality did not change. False Discovery Rate (FDR), estimated by q-value, is the proportion of significant changes that are false positives. The q-value for each marker was derived using the method proposed by Benjamini & Hockberg (Benjamini and Hockberg, 2000, J. Behav. Educ. Statist. 25:60-83). All analyses were carried out using R, version 2.4 (R Development Core Team (2006). R: A language and environment for statistical computing. R Foundation for Statistical Computing, Vienna, Austria. ISBN 3-900051-07-0, URL http://www.R-project.org.)

We first compared the average (median) between COPD rapid decliners and controls for each one of the 89 plasma markers tested. Many small but significant (p < 0.05) differences were observed between these two groups (Table 3). False Positive Rate (FPR), estimated by p-value, is the proportion of false positives among all the markers that in reality did not change. False Discovery Rate (FDR), estimated by q-value, is the proportion of significant changes that are false positives. A positive fold change value represents an increase over healthy control, and a negative value represents a decrease. In terms of volcanic fold change, the strongest differences were seen with interleukin-4 (IL-4; 4.2-fold increase in rapid decliners), eotaxin (2.6-fold increase), interleukin-7 (IL-7; 2.2-fold increase) and haptoglobin (2.0-fold increase). The statistically most significant differences were observed for IL-4, monocyte chemoattractant protein (MCP-1), soluble vascular cell adhesion molecule-1 (sVCAM-1) and eotaxin with p value (false positive rates) less than 0.1% and q value (false discovery rate) less than 1% (Figure 1). In contrast, univariate comparison of slow COPD decliners to controls revealed fewer and less significant differences (Table 4). Only 4 markers had a p value below 0.05, and in all four cases the false discovery rate was quite elevated (>50%). Finally, some interesting differences were seen when comparing slow and rapid COPD decliners (Table 5). Interleukin-4 (IL-4), interleukin-5 (IL-5), monocyte chemoattractant protein-1 (MCP-1), macrophage-derived chemokine (MDC) and tissue factor were present at significantly higher levels in rapid decliners compared to slow decliners (p < 0.05). Cancer antigen 19.9, immunoglobulin type A (IgA), insulin, macrophage inflammatory protein 1 alpha (MIP-1) and soluble vascular cell adhesion molecule 1 (sVCAM-1) were present at significantly lower levels in rapid decliners compared to slow decliners.

**Table 3: Differences in plasma markers between COPD rapid decliners and healthy controls as determined by univariate analysis.**

| **Marker** | **Fold change** | **p value (FPR)** | **q value (FDR)** |
|---|---|---|---|
| Alpha-1-antitrypsin | 1.11 | 0.0238 | 0.062 |
| Alpha fetoprotein | 1.38 | 0.0498 | 0.094 |
| Apolipoprotein A1 | 1.38 | 0.0020 | 0.017 |
| Apolipoprotein H | 1.15 | 0.0029 | 0.019 |
| Carcinoembryonic antigen | 1.75 | 0.0022 | 0.017 |
| Eotaxin | 2.64 | 0.0007 | 0.008 |
| Factor VII | 1.16 | 0.0448 | 0.091 |
| Fibrinogen | 1.18 | 0.0231 | 0.062 |
| GM-CSF | 1.51 | 0.0061 | 0.026 |
| Haptoglobin | 2.02 | 0.0115 | 0.034 |
| IL-10 | 1.54 | 0.0116 | 0.034 |
| IL-13 | 1.69 | 0.0086 | 0.031 |
| IL-1 alpha | 1.16 | 0.0336 | 0.079 |
| IL-3 | 1.46 | 0.0496 | 0.094 |
| IL-4 | 4.21 | <0.0001 | 0.000 |
| IL-5 | 1.59 | 0.0041 | 0.023 |
| IL-7 | 2.16 | 0.0044 | 0.023 |
| IL-8 | 1.20 | 0.0398 | 0.088 |
| MCP-1 | 1.51 | <0.0001 | 0.001 |
| Serum amyloid P | 1.28 | 0.0049 | 0.023 |
| Tissue factor | 1.19 | 0.0410 | 0.088 |
| TNF-RII | -1.19 | 0.0071 | 0.028 |
| Thrombopoietin | 7.55 | 0.0117 | 0.034 |
| sVCAM-1 | -1.20 | 0.0002 | 0.003 |
| VEGF | 1.18 | 0.0301 | 0.075 |

**Table 4: Differences in plasma markers between COPD slow decliners and healthy controls as determined by univariate analysis.**

| **Marker** | **Fold change** | **p value (FPR)** | **q value (FDR)** |
|---|---|---|---|
| Apolipoprotein H | 1.16 | 0.0230 | 0.696 |
| Cancer antigen 19.9 | 2.29 | 0.0078 | 0.563 |
| Eotaxin | 1.68 | 0.0358 | 0.696 |
| VEGF | 1.11 | 0.0545 | 0.696 |

**Table 5: differences in plasma markers between COPD rapid and slow decliners as determined by univariate analysis.**

| **Marker** | **Fold change** | **p value (FPR)** | **q value (FDR)** |
|---|---|---|---|
| Cancer antigen 19.9 | -1.43 | 0.0355 | 0.300 |
| IgA | -1.57 | 0.0120 | 0.157 |
| IL-4 | 1.27 | 0.0008 | 0.054 |
| IL-5 | 1.48 | 0.0139 | 0.157 |
| Insulin | -5.86 | 0.0165 | 0.160 |
| MCP-1 | 1.44 | 0.0026 | 0.089 |
| MDC | 1.28 | 0.0422 | 0.300 |
| MIP-1 alpha | -1.26 | 0.0468 | 0.300 |
| Tissue factor | 1.32 | 0.0125 | 0.157 |
| sVCAM-1 | -1.31 | 0.0043 | 0.098 |

*Multivariate analysis for the identification of signatures -* Mult-analyte panels (signatures) that provide optimal separation between COPD rapid decliners and healthy controls, COPD slow decliners and healthy controls, and COPD rapid and COPD slow decliners were determined using a forward selection procedure with Linear Discriminant Analysis (Venables, W.N. & Ripley, B.D., (2002). Modern Applied Statistics, Fourth Edition. Springer). This analysis measured the distance from each point in the data set to each group's multivariate mean (called a centroid) and classified the point to the closest group. The distance measure sued was the Mahalanobis distance which takes into account the variances and covariances between the variables. Representative multi-analyte panels (signatures) are shown in Table 6 along with their predictive performance. The predictive performance of these markers to separate COPD from control for hold-out datasets was investigated using fifty replications of 5-fold cross-validation. This entailed dividing up the data randomly into five subgroups, leaving each group out at a time, building a model on the remaining four subgroups, using this fitted model to predict the disease classification in the fifth subgroup, repeating this for all five groups and then averaging the results. This analysis was repeated fifty times to generate a reliable estimate of the overall accuracy along with the sensitivity and specificity of the biomarker signatures. These analyses were based on 20 controls and 14 or 26 COPD subjects and carried out using R, version 2.4 (R Development Core Team (2006). R: A language and environment for statistical computing. R Foundation for Statistical Computing, Vienna, Austria. ISBN 3-900051-07-0, URL http://www.R-project.org.).

**Table 6: Summary of representative multi-analyte panels (signatures), along with their predictive performance, as identified by multivariate analysis.**

| **Groups Compared** | **Accuracy** | **Signature Component** |
|---|---|---|
| RAPID decliners (n=26) | 90% | Apolipoprotein H |
| vs. | | CD40 |
| Healthy smokers (n=20) | | Haptoglobin |
| (Year 1) | | IL-8 |
| | | MCP-1 |
| | | TNF-RII |
| RAPID decliners (n=9) | 93% | |
| vs. | | |
| Healthy smokers (n=5) (Year 2) | | |
| SLOW decliners (n=14) | 94% | Apolipoprotein CIII |
| vs. | | CD40 |
| Healthy smokers (n=20) | | GM-CSF |
| | | Haptoglobin |
| | | IgA |
| | | MIP1-alpha |
| | | Tissue Factor |
| | | TNF-alpha |
| RAPID decliners (n=26) | 92% | Alpha-1 antitrypsin |
| vs. | | CRP |
| SLOW decliners (n=14) | | Fibrinogen |
| | | GM-CSF |
| | | IL-4 |
| | | MDC |
| | | Tissue Factor |
| | | TNF-RII |
| | | VCAM-1 |

Since none of the plasma markers were capable on their own to clearly distinguish COPD patients from controls, we performed a multivariate analysis of the data using the Linear Discriminant Analysis method (LDA). This method identified a group of plasma markers (signature 1) capable of accurately distinguishing COPD rapid decliners from controls (healthy smokers) (Figure 2A). Figure 2A is an output from a linear discriminant analysis (LDA) of a two-dimensional representation using principal components of a six-marker signature for separating COPD rapid decliners from healthy controls. The distance measure used is the Mahalanobis distance, which takes into account the variance and covariance between the variables. Each multivariate mean is a labeled circle. The size of the circle corresponds to a 95% confidence limit for the mean. Groups that are significantly different tend to have non-intersecting circles. Signature 1 is composed of the following 6 markers: apolipoprotein H, CD40, haptoglobin, interleukin-8 (IL-8), monocyte chemoattractant protein-1 (MCP-1) and tumor necrosis factor receptor II (TNF-RII). Univariate analysis of these markers revealed significant differences (p < 0.05; Table 3) except for CD40 (1.15-fold, p < 0.5). Fifty replicates of 5-fold cross-validation revealed that signature 1 can correctly identify that a plasma sample is from a COPD rapid decliner in 93% of the cases (sensitivity) and that a plasma sample is from a healthy subject in 86% of the cases (specificity) for an overall accuracy to distinguish between the two groups of approximately 90%.

We next wanted to verify the longitudinal stability of signature 1. Blood samples from 9 of the 26 COPD rapid decliners and 5 of the 20 healthy controls were collected approximately 1 year after the initial samples were used to identify signature 1. This signature correctly identified all 9 COPD rapid decliners and 4 out of 5 healthy controls for an overall accuracy of approximately 93% (Table 6).

Using the LDA method, we also identified a second group of plasma markers (signature 2) capable of accurately distinguishing COPD slow decliners from healthy controls (Figure 2). Figure 2 is an output from a linear discriminant analysis (LDA) of a two-dimensional representation using principal components of an eight-marker signature for separating COPD slow decliners from healthy controls. Signature 2 is composed of the following 8 markers: apolipoprotein CIII, CD40, granulocyte-macrophage colony stimulating factor (GM-CSF), haptoglobin, immunoglobulin A (IgA), macrophage inflammatory protein 1 alpha (MIP-1 ), tissue factor and tumor necrosis factor-alpha (TNF- ). Fifty replicates of 5-fold cross-validation revealed that signature 2 has 91 % sensitivity and 96% specificity for an overall accuracy to distinguish slow decliners from control of approximately 94% (Table 6).

Finally, we identified a third group of plasma markers (signature 3) capable of accurately distinguish COPD rapid from slow decliners (Figure 2C). Figure 2C is an output from a linear discriminant analysis (LDA) of a two-dimensional representation using principal components of a nine-marker signature for separating COPD slow and rapid decliners. Signature 3 is composed of the following 9 markers: alpha-1 antitrypsin, C-reactive protein (CRP), fibrinogen, granulocyte-macrophage colony stimulating factor (GM-CSF), interleukin-4 (IL-4), macrophage-derived chemokine (MDC), tissue factor, tumor necrosis factor receptor II (TNF-RII) and soluble vascular cell adhesion molecule-1 (sVCAM-1). Fifty replicates of 5-fold cross-validation revealed that signature 3 can correctly identify that a plasma sample is from a COPD rapid decliner in 95% of the case and that a plasma sample is from a COPD slow decliner in 86% of the case for an overall accuracy to distinguish between the two groups of approximately 92% (Table 6).

Table 7 lists representative composites of biomarkers from LDA analysis that provide impressive levels of sensitivity and specificity. LDA for assessing the performance of the various composites of markers was performed using a contributed library within R (Venables, W.N. & Ripley, B.D., (2002). Modern Applied Statistics, Fourth Edition. Springer, New York). The 5-fold cross-validation for obtaining reliable estimates of the performance metrics of the biomarker composites was performed using a contributed library within R (Andrea Peters and Torsten Hothorn, 2004, Improved Predictors, R package version 0.8-3).

**Table 7: Summary of the percent accuracy ("Acc"), sensitivity ("Sen") and specificity ("Spe") as determined by Linear Discrininant Analysis (LDA) for each of the three biomarker signatures ("optimal-LDA") disclosed in the present application, as well as for subsets of these optimal signatures containing the best of 3, 4 and/or 5 of the biomarkers.**

| | | % **Acc** | **% Sen** | % **Spe** |
|---|---|---|---|---|
| | **Signature 1** | | | |
| **CTL vs COPD-Rapid** | | | | |
| Optimal-LDA (6 markers) | Apolipoprotein H, CD40, Haptoglobin, IL-8, MCP-1, TNF-RII | 90.1 | 93.1 | 86.2 |
| best 3 out of above | Apolipoprotein H, MCP-1, TNF-RII | 89 | 90.6 | 87 |
| best 4 out of above | Apolipoprotein H, CD40, MCP-1, TNF-RII | 88.2 | 91.4 | 84.1 |

| | **Signature 2** | | | |
|---|---|---|---|---|
| **CTL vs COPD-Slow** | | | | |
| Optimal-LDA (8 markers) | Apolipoprotein CIII, CD40, GM-CSF, Haptoglobin, IgA, MIP-1 , Tissue factor, TNF | 94.1 | 90.7 | 96.4 |
| best 3 out of above | IgA, MIP-1 , Tissue factor | 76.1 | 62 | 86 |
| best 4 out of above | GM-CSF, IgA, MIP-1 , Tissue factor | 78.3 | 63.3 | 88.9 |
| best 5 out of above | Apolipoprotein CIII, GM-CSF, IgA, MIP-1 , Tissue factor | 83.3 | 68.4 | 93.8 |

| | **Signature 3** | | | |
|---|---|---|---|---|
| **COPD-Rapid vs COPD-Slow** | | | | |
| Optimal-LDA (9 markers) | Alpha1-antitrypsin, CRP, Fibrinogen, GM-CSF, IL-4, MDC, Tissue factor, sVCAM-1, TNF-RII | 91.8 | 94.9 | 86.1 |
| best 3 out of above | MDC, Tissue factor, sVCAM-1 | 80.3 | 89.4 | 63.4 |
| best 4 out of above | IL-4, MDC, Tissue factor, sVCAM-1 | 85.4 | 92.9 | 71.3 |
| best 5 out of above | GM-CSF, IL-4, MDC, Tissue factor, sVCAM-1 | 85.9 | 92.9 | 73 |

*Discussion -* In this study, we identified plasma proteins which levels are statistically different in patients with COPD compared to healthy controls. Out of the 89 plasma markers evaluated, 25 were statistically different between rapid decliners and controls (p<0.05), while only 4 markers were different between slow decliners and controls (p< 0.05). These univariate analyses revealed that there are actually more statistically significant differences between rapid and slow decliners (10 markers with p<0.05) than there are between slow decliners and controls (only 4 markers with p<0.05). To our knowledge, this is the first study providing such an extensive serological differentiation between slow and rapid manifestations of this disease.

Some of the markers showing differences between rapid decliners and controls have been previously reported to be modulated in the blood of non-exacerbated COPD patients, such as 1-antitrypsin (Aldonyte et al., 2004, COPD 1:155-164), eotaxin (Aldonyte et al., 2004, *supra;* Janhz-Rozyk et al., 2000, Pol. Merkur. Lekarski. 9:649-652), fibrinogen (Gan et al., 2004, Thorax 59:574-580), IL-4 (Zhang et al., 1999, J. Tongji Med. Univ. 19:15-19), IL-8, MCP-1 and VEGF (Pinto-Plata et al., 2007, Thorax 62:595-601). Out of the 25 markers significantly modulated in rapid decliners compared to controls, only two of them are decreased in rapid decliners: soluble receptor II for TNF- (sTNFRII) and the soluble form of vascular cell adhesion molecule-1 (sVCAM-1). Interestingly, these are both anti-inflammatory proteins. sTNFRII traps the pro-inflammatory protein TNF- (Carpentier et al., 2004, Curr. Med. Chem. 11:2205-2212) while sVCAM-1 interferes with the adhesion of VCAM-1-bearing leukocytes to endothelial cells expressing the VCAM-1 ligand, 4 1 integrin (Foster, 1996, J. Allergy Clin. Immunol. 98:S270-S277).

In the panel of markers significantly increased in the rapid decliners over controls, it is interesting to note the presence of chemoattractants such as GM-CSF, IL-8 and MCP-1 known to modulate the activity of neutrophils and macrophages, two cell types implicated in COPD (Barnes, 2004, Pharmacol. Rev. 56:515-548). It is even more interesting to note in that list the presence of several mediators associated to Th2/Tc2 phenotype such as IL-4, IL-5, IL-10, IL-13 and eotaxin. This is in agreement with a recent study reporting that T-lymphocytes present in the lung of COPD patients produce significantly more IL-4, IL-10 and IL-13 than T-lymphocytes from healthy smokers (Barcelo et al., 2006, Clin. Exp. Immunol. 145:474-479). This observation supports a hypothesis suggesting an overlap between asthma and COPD in more severe form of the diseases (Jeffery, Peter K. "Lymphocytes, Chronic Bronchitis and Chronic Obstructive Pulmonary Disease." Chronic Obstructive Pulmonary Disease: Pathogenesis to Treatment: Novartis Foundation Symposium, Volume 234. Eds. Derek Chadwick & Jamie A. Goode. Chichester: Wiley, 2001. 149-168).

COPD, like many other chronic diseases, is thought to be a highly heterogeneous disease with phenotypic expressions influenced by genetic and environmental factors. This leads to the expected intra-group variability observed for each plasma markers when performing univariate analysis. Similar variability after univariate analysis of markers in serum from COPD and controls was recently described by another group (Pinto-Plata et al., 2007, *supra*)*.* In this context, each individual plasma marker has limited potential to accurately distinguish between samples from healthy individuals and patients with either slow or rapid forms of COPD. The combination of multiple markers in a multivariate analysis takes in consideration this intra-group heterogeneity. Multivariate analysis led to the identification of a 6-marker signature capable of distinguishing rapid decliners from healthy controls with about 90% accuracy. Importantly, similar accuracy was obtained with these same 6 markers when analyzing samples obtained approximately 1 year later from the same cohort, suggesting that the signature is longitudinally stable and thus of potential clinical utility. The signature is composed of chemoattractants for neutrophils and monocytes such as IL-8 and MCP-1 with putative roles in the inflammation that characterizes COPD (Barnes, 2004, *supra*)*;* the circulating soluble form of a surface glycoprotein named CD40 previously reported to be elevated in patients with chronic renal failure (Schwabe et al., 1999, Clin. Exp. Immunol. 117:153-158), chronic liver disease (Schmilovitz-Weiss et al., 2004, Apoptosis 9:205-210), Alzheimer's disease (Mocali et al., 2004, Exp. Gerontol. 39:1555-1561) and Systemic Sclerosis (Komura et al., 2007, J. Rheumatol. 34:353-358); haptoglobin, an acute phase protein with antioxidant and anti-inflammatory properties (Zvi and Levy, 2006, Clin. Lab. 52:29-35); the anti-inflammatory sTNFRII; and, apolipoprotein H, a plasma glycoprotein implicated in a variety of physiological pathways including blood coagulation, haemostasis and the production of anti-phospholipid antibodies (McNeil et al., 1990, Proc. Natl. Acad. Sci. USA 87:4120-4124).

sCD40 and haptoglobin are also part of a group of 8 plasma markers capable of distinguishing slow decliners from controls with about 94% accuracy. This signature also includes 3 other cytokines/chemokines: GM-CSF, a regulator of neutrophil survival and activity found at elevated levels in bronchoalveolar fluid from stable and exacerbated COPD patients (Balbi et al., 1997, Eur. Respir. J. 10:846-850); MIP1- , a chemoattractant for neutrophils and monocytes (Barnes, 2004, *supra*); and, TNF- , a cytokine implicated in COPD (Barnes, 2004, *supra*) and found at higher levels in the blood of stable COPD patients (Gan et al., 2994, *supra*)*.* The COPD slow decliner signature is completed by apolipoprotein CIII, a modulator of lipoprotein metabolism (Shachter, 2001, Curr. Opin. Lipidol. 12:297-304); IgA, a subtype of circulating immunoglobulin that has been previously associated with anti-phospholipid antibodies (Staub et al., 2006, Autoimmun. Rev. 6:104-106); and, soluble tissue factor (sTF), a coagulating factor that has been found to be elevated in patients with anti-phospholipid syndrome characterized by the presence of anti-phospholipid antibodies and thromboembolic complications.

The rapid versus slow COPD decliner signature is composed of 9 plasma markers. Three of those markers, -antitrypsin, C-reactive Protein (CRP) and fibrinogen, have been extensively reported to be modulated in COPD patients (Gan et al., 2004, *supra;* Ranes and Stoller, 2005, Semin. Respir. Crit. Care Med. 26:154-166). Three other markers are cytokines/chemokines: GM-CSF; IL-4; and, macrophage-derived chemokine (MDC). GM-CSF is also included in the slow decliner signature and has been discussed above. IL-4 is the most significantly increased marker in rapid decliners compared to slow decliners and controls in the univariate analysis. MDC (CCL22) has been shown to be up-regulated by IL-4 and plays an important role in the recruitment of Th2 cells to inflammatory sites (Gan et al., 2004, *supra;* Yamashita and Kuroda, 2002, Crit. Rev. Immunol. 22:105-114). The signature is completed with sTF, sTNFRII and sVCAM-1 described above.

Attempts to identify a single signature that would distinguish controls, slow and rapid COPD decliners did not yield accuracies above 82%. The need for distinct signatures to accurately distinguish rapid or slow COPD decliners from controls and the fact that a signature can be found that accurately distinguishes rapid from slow COPD decliners suggest the existence of fundamental biochemical differences linked to the rate of lung function decline in COPD. To our knowledge, this is the first study describing plasma markers differentiating slow from rapid declining form of COPD.

The present invention is further defined by the following clauses:
1. A computer-implemented method for classifying a test sample obtained from a human subject, comprising:
   (a) obtaining a dataset associated with said test sample, wherein said obtained dataset comprises quantitative data for at least three protein markers selected from a multi-analyte panel selected from the group consisting of:
      (i) apolipoprotein H, CD40, haptoglobin, interleukin-8 (IL-8), monocyte chemoattractant protein-1 (MCP-1) and tumor necrosis factor receptor II (TNF-RII);
      (ii) apolipoprotein CIII, CD40, granulocyte-macrophage colony stimulating factor (GM-CSF), haptoglobin, immunoglobulin A (IgA), macrophage inflammatory protein 1alpha (MIP-1 ), tissue factor and tumor necrosis factor-alpha (TNF- ); and,
      (iii) alpha-1 antitrypsin, C-reactive protein (CRP), fibrinogen, granulocyte-macrophage colony stimulating factor (GM-CSF), interleukin-4 (IL-4), macrophage-derived chemokine (MDC), tissue factor, tumor necrosis factor receptor II (TNFRII) and soluble vascular cell adhesion molecule 1 (sVCAM-1);
   (b) inputting said obtained dataset into an analytical process on a computer that compares said obtained dataset against one or more reference datasets; and,
   (c) classifying said test sample according to the output of said analytical process, wherein said classification is selected from the group consisting of a chronic obstructive pulmonary disease (COPD) classification and a healthy classification.
2. The method of clause 1, further comprising a step of, after classification of said test sample, either (a) transforming said classification information either to assess efficacy of a drug in a clinical trial, to diagnose COPD in said subject, to assess extent of COPD progression in said subject, or to develop a COPD treatment plan for said subject; or, (b) treating said subject against COPD.
3. The method of clause 1 or 2, wherein said test sample is selected from the group consisting of blood, plasma and serum.
4. The method of clause 3, wherein said analytical process comprises use of a predictive model which comprises the one or more reference datasets.
5. The method of clause 4, wherein a COPD classification represents either a rapidly declining COPD classification or a slowly declining COPD classification.
6. The method of clause 5, wherein said one or more reference datasets comprise quantitative data obtained from one or more human subjects selected from a group consisting of healthy subjects, subjects diagnosed with rapidly declining COPD, and subjects diagnosed with slowly declining COPD.
7. The method of clause 6, wherein said at least three protein markers comprise apolipoprotein H, MCP-1 and TNF-RII.
8. The method of clause 7, wherein said obtained dataset comprises quantitative data from apolipoprotein H, CD40, haptoglobin, IL-8, MCP-1 and TNF-RII.
9. The method of clause 6, wherein said at least three protein markers comprise IgA, MIP-1 and tissue factor.
10. The method of clause 9, wherein said obtained dataset comprises quantitative data from apolipoprotein CIII, CD40, GM-CSF, haptoglobin, IgA, MIP-1 , tissue factor and TNF- .
11. The method of clause 6, wherein said at least three protein markers comprises MDC, tissue factor and sVCAM-1.
12. The method of clause 11, wherein said obtained dataset comprises quantitative data from alpha-1 antitrypsin, CRP, fibrinogen, GM-CSF, IL-4, MDC, tissue factor, TNF-RII and sVCAM-1.
13. The method of clause 1 or 2, wherein said analytical process comprises using either a Linear Discriminant Analysis (LDA) model, a support vector machine classification algorithm, a recursive feature elimination model, a prediction analysis of microarray model, a Logistic Regression model, a CART algorithm, a FlexTree algorithm, a LART algorithm, a random forest algorithm, a MART algorithm, or Machine Learning algorithms.
14. The method of clause 13, wherein said analytical process comprises using a LDA model comprising terms selected to provide a quality metric greater than 75%.
15. The method of clause 14, wherein said quality metric is accuracy.
16. The method of clause 15, wherein the LDA model is adjusted to provide at least one of sensitivity or specificity of at least 70%.
17. The method of clause 4, wherein said predictive model has a quality metric of at least 75% for classification.
18. The method of clause 17, wherein said predictive model has a quality metric of at least 90% for classification.
19. The method of clause 17, wherein said quality metric is accuracy.
20. The method of clause 19, wherein the limits of said predictive model are adjusted to provide at least one of sensitivity or specificity of at least 70%.
21. A kit for classifying a test sample obtained from a human subject, comprising reagents for detecting at least three protein markers selected from a multi-analyte panel selected from the group consisting of:
   (a) apolipoprotein H, CD40, haptoglobin, interleukin-8 (IL-8), monocyte chemoattractant protein-1 (MCP-1) and tumor necrosis factor receptor II (TNF-RII);
   (b) apolipoprotein CIII, CD40, granulocyte-macrophage colony stimulating factor (GM-CSF), haptoglobin, immunoglobulin A (IgA), macrophage inflammatory protein 1alpha (MIP-1 ), tissue factor and tumor necrosis factor-alpha (TNF- ); and,
   (c) alpha-1 antitrypsin, C-reactive protein (CRP), fibrinogen, granulocyte-macrophage colony stimulating factor (GM-CSF), interleukin-4 (IL-4), macrophage-derived chemokine (MDC), tissue factor, tumor necrosis factor receptor II (TNFRII) and soluble vascular cell adhesion molecule 1 (sVCAM-1);
   wherein said classification is selected from the group consisting of a chronic obstructive pulmonary disease (COPD) classification and a healthy classification.
22. The kit of clause 21, wherein said reagents are antibodies.

## Claims

1. A computer-implemented method for classifying a test sample obtained from a human subject, comprising:
(a) obtaining and analyzing a test sample from the human subject to obtain a dataset associated with said test sample, wherein said obtained dataset comprises quantitative data for a set of at least four protein markers all of which are selected from one of multi-analyte panels (i), (ii) or (iii):
(i) alpha-1 antitrypsin, C-reactive protein (CRP), fibrinogen, granulocyte-macrophage colony stimulating factor (GM-CSF), interleukin-4 (IL-4), macrophage-derived chemokine (MDC), tissue factor, tumor necrosis factor receptor II (TNFRII), and soluble vascular cell adhesion molecule 1 (sVCAM-1);
(ii) apolipoprotein H, CD40, haptoglobin, interleukin-8 (IL-8), monocyte chemoattractant protein-1 (MCP-1), and tumor necrosis factor receptor II (TNF-RII); and
(iii) apolipoprotein CIII, CD40, granulocyte-macrophage colony stimulating factor (GM-CSF), haptoglobin, immunoglobulin A (IgA), macrophage inflammatory protein 1alpha (MIP-1α), tissue factor, and tumor necrosis factor-alpha (TNF-α); and
(b) inputting said obtained dataset into an analytical process on a computer that compares said obtained dataset against one or more reference datasets; and,
(c) classifying said test sample according to the output of said analytical process, wherein said classification is selected from the group consisting of a rapidly declining chronic obstructive pulmonary disease (COPD) classification, a slowly declining COPD classification, and a healthy classification.

2. The method of claim 1, wherein said test sample is selected from the group consisting of blood, plasma and serum.

3. The method of any previous claim, wherein said obtained dataset comprises quantitative data for four or more protein markers selected from the group consisting of:
apolipoprotein H, CD40, haptoglobin, interleukin-8 (IL-8), monocyte chemoattractant protein-1 (MCP-1) and tumor necrosis factor receptor II (TNF-RII);
and wherein classifying said test sample according to the output of said analytical process further includes a classification selected from a rapid declining COPD classification and a healthy classification.

4. The method of claim 3, wherein the four or more markers comprise Apolipoprotein H, CD40, MCP-1, and TNF-RII.

5. The method of any previous claim, wherein said obtained dataset comprises quantitative data for four or more protein markers selected from the group consisting of:
apolipoprotein CIII, CD40, granulocyte-macrophage colony stimulating factor (GM-CSF), haptoglobin, immunoglobulin A (IgA), macrophage inflammatory protein 1 alpha (MIP-1α), tissue factor, and tumor necrosis factor-alpha (TNF-α));
and wherein classifying said test sample according to the output of said analytical process further includes a classification selected from a slowly declining COPD classification and a healthy classification.

6. The method of claim 5, wherein the four or more markers comprise GM-CSF, IgA, MIP-1a, and Tissue factor.

7. The method of any previous claim, wherein said obtained dataset comprises quantitative data for four or more protein markers selected from the group consisting of:
macrophage-derived chemokine (MDC), tissue factor, soluble vascular cell adhesion molecule 1 (sVCAM-1 ), alpha-1 antitrypsin, C-reactive protein (CRP), fibrinogen, granulocyte-macrophage colony stimulating factor (GM-CSF), interleukin-4 (IL-4) or tumor necrosis factor receptor II (TNF-RII);
and wherein classifying said test sample according to the output of said analytical process further includes a classification selected from a rapidly declining COPD classification and a slowly declining COPD classification.

8. The method of claim 7, wherein the four or more markers comprise IL-4, MDC, Tissue factor, and sVCAM-1.

9. The method of claim 1, wherein the obtained dataset comprises at least four protein markers from each of two or more different multi-analyte panels selected from multi-analyte panels (i), (ii), and (iii); and
wherein said classifying comprises classifying said test sample according to the output of said analytical process for each of the two or more different multi-analyte panels in the data set, wherein said classification includes two or more of a healthy classification versus a rapidly declining COPD classification, a healthy versus slowly declining COPD classification, and a rapidly declining COPD classification versus slowly declining COPD classification.

10. A kit for classifying a test sample obtained from a human subject, comprising reagents for detecting at least four protein biomarkers selected from one of multi-analyte panels (i), (ii), or (iii):
(i) alpha-1 antitrypsin, C-reactive protein (CRP), fibrinogen, granulocyte-macrophage colony stimulating factor (GM-CSF), interleukin-4 (IL-4), macrophage-derived chemokine (MDC), tissue factor, tumor necrosis factor receptor II (TNFRII) and soluble vascular cell adhesion molecule 1 (sVCAM-1);
(ii) apolipoprotein H, CD40, haptoglobin, interleukin-8 (IL-8), monocyte chemoattractant protein-1 (MCP-1) and tumor necrosis factor receptor II (TNF-RII); and
(iii) apolipoprotein CIII, CD40, granulocyte-macrophage colony stimulating factor (GM-CSF), haptoglobin, immunoglobulin A (IgA), macrophage inflammatory protein 1alpha (MIP-1α), tissue factor and tumor necrosis factor-alpha (TNF-α).

11. A method for classifying a test sample obtained from a human subject, comprising:
(a) obtaining and analyzing a test sample from the human subject to obtain a dataset associated with said test sample, wherein said obtained dataset comprises quantitative data for a set of at least four protein markers all of which are selected from one of multi-analyte panels (i), (ii) or (iii):
(i) alpha-1 antitrypsin, C-reactive protein (CRP), fibrinogen, granulocyte-macrophage colony stimulating factor (GM-CSF), interleukin-4 (IL-4), macrophage-derived chemokine (MDC), tissue factor, tumor necrosis factor receptor II (TNFRII), and soluble vascular cell adhesion molecule 1 (sVCAM-1);
(ii) apolipoprotein H, CD40, haptoglobin, interleukin-8 (IL-8), monocyte chemoattractant protein-1 (MCP-1), and tumor necrosis factor receptor II (TNF-RII); and
(iii) apolipoprotein CIII, CD40, granulocyte-macrophage colony stimulating factor (GM-CSF), haptoglobin, immunoglobulin A (IgA), macrophage inflammatory protein 1alpha (MIP-1α), tissue factor, and tumor necrosis factor-alpha (TNF-α); and
(b) inputting said obtained dataset into an analytical process on a computer that compares said obtained dataset against one or more reference datasets; and,
(c) classifying said test sample according to the output of said analytical process, wherein said classification is selected from the group consisting of a rapidly declining COPD classification, a slowly declining COPD classification, and a healthy classification.

12. The method of claim 11, wherein said obtained dataset comprises quantitative data for four or more protein markers selected from the group consisting of:
macrophage-derived chemokine (MDC), tissue factor, soluble vascular cell adhesion molecule 1 (sVCAM-1 ), alpha-1 antitrypsin, C-reactive protein (CRP), fibrinogen, granulocyte-macrophage colony stimulating factor (GM-CSF), interleukin-4 (IL-4) or tumor necrosis factor receptor II (TNF-RII);
and wherein classifying said test sample according to the output of said analytical process further includes a classification selected from a rapidly declining COPD classification and a slowly declining COPD classification.

13. The method of claim 11, wherein the obtained dataset comprises at least four protein markers from two or more different multi-analyte panels selected from multi-analyte panels (i), (ii), and (iii); and
wherein said classifying comprises classifying said test sample according to the output of said analytical process for each of the two or more different multi-analyte panels in the data set, wherein said classification includes two or more of a healthy classification versus a rapidly declining COPD classification, a healthy versus slowly declining COPD classification, and a rapidly declining COPD classification versus slowly declining COPD classification.

14. The method of claim 11, wherein said analyzing is conducted using a biochip.

15. A biochip for detecting least four protein markers from one multi-analyte panel selected from multi-analyte panels (i), (ii) or (iii):
(i) alpha-1 antitrypsin, C-reactive protein (CRP), fibrinogen, granulocyte-macrophage colony stimulating factor (GM-CSF), interleukin-4 (IL-4), macrophage-derived chemokine (MDC), tissue factor, tumor necrosis factor receptor II (TNFRII), and soluble vascular cell adhesion molecule 1 (sVCAM-1);
(ii) apolipoprotein H, CD40, haptoglobin, interleukin-8 (IL-8), monocyte chemoattractant protein-1 (MCP-1), and tumor necrosis factor receptor II (TNF-RII); and
(iii) apolipoprotein CIII, CD40, granulocyte-macrophage colony stimulating factor (GM-CSF), haptoglobin, immunoglobulin A (IgA), macrophage inflammatory protein 1alpha (MIP-1α), tissue factor, and tumor necrosis factor-alpha (TNF-α).
